# EUROPEAN PATENT APPLICATION

(11) **EP 4 552 641 A2**
(43) Date of publication of application: **14.05.2025**
(21) Application number: 25151222.4
(22) Date of filing: 08.05.2017
(51) Int. Cl.: A61K 31/4196

(54) **IMPROVED DRUG FORMULATIONS**

(30) Priority: 09.05.2016 US 201662333482 P; 11.05.2016 US 201662334576 P
(62) Divisional of application: 17796620.7
(71) Applicant: AustinPx, LLC, Georgetown, TX 78626 (US)
(72) Inventor: Miller, Dave A., 3749 Pine Needle Cir., 78681 (US); Ellenberger, Daniel J., 8910 Marybank Dr., 78750 (US); Schilling, Sandra U., 709 E. 44th St. Apt. A, 78751 (US)
(74) Representative: Rückerl, Florian

(57) **Abstract**

The disclosure provides for improved pharmaceutical compositions containing an active pharmaceutical ingredient and a non-polymeric lubricant and methods of manufacturing the same. In particular, the compositions are prepared using thermal processing or solvent sprying and provide improved properties as well as more efficient methods of manufacture.

## Description

### BACKGROUND

This application claims benefit of priority to U.S. Provisional Application Serial No. 62/333,482, filed May 9, 2016, and U.S. Provisional Application Serial No. 62/334,576, filed May 11, 2016, the entire contents of both applications being hereby incorporated by reference.

### BACKGROUND

### 1. Field

The present disclosure relates in general to the field of pharmaceutical preparation and manufacturing, and more particularly, pharmaceutical formulations of poorly soluble drugs that include a lubricant disposed within an amorphous solid dispersion.

### 2. Description of Related Art

The beneficial applications of many potentially therapeutic molecules is often not fully realized either because they are abandoned during development due to poor pharmacokinetic profiles, or because of suboptimal product performance. Alternatively, even if produced, the cost associated with formulating such molecules may create barriers to their widespread use. Problems with formulation are often due to poor solubility, resulting in poor bioavailability, increased expense, and ultimately termination of the product's development. In recent years, the pharmaceutical industry has begun to rely more heavily on formulational methods for improving drug solubility. Consequently, advanced formulation technologies aimed at enhancing the dissolution properties of poorly water soluble drugs are becoming increasingly important to modern drug delivery.

In pharmaceutical processing, lubricants are essential components of a drug formula since lubrication is often required to ensure the success of pharmaceutical manufacturing. In particular, in the pharmaceutical industry, the application of lubrication or tribology in drug development has become increasingly important for developing a successful manufacturing process. For pharmaceutical operations (*e*.*g*., as blending, roller compaction, tablet manufacturing, capsule-filling), lubrication is essential in order to reduce the friction between the surfaces of manufacturing equipment and that of organic solids as well as to ensure the continuation of an operation. Pharmaceutical lubricants are added to tablet and capsule formulations to improve the processing properties of formulations. Even though used in small amounts, lubricants play an important role. For example, they help decrease friction at the interface between a tablet's surface and a die wall during ejection so that the wear on punches and dies are reduced. They can prevent sticking of tablets to punch faces as well as sticking of capsules to dosators and tamping pins. And lubricants can improve the flowability of blends and aid unit operations.

However, the use of lubricants is not without its limitations, and as such, conventional amorphous dispersion techniques would not typically include a lubricant as a processing aid. For example, it is suspected that spray-drying an amorphous composition containing lubricant would be very challenging due to the insoluble nature of crystalline lubricants. Thus, in the case of hot-melt extrusion, other additives/techniques are typically applied. Crystalline, non-polymeric, poorly soluble lubricants typically would not have been considered as a solubility enhancer due to their hydrophobic/water-insoluble nature. As such, they would not be expected to improve drug solubility as they would not be dissolved in solution. Studies have in fact shown that inclusion of these agents in a crystalline form, in final tablet or capsule formulation, often hinder solubility/bioavailability. Also, in the case of spray-drying, a lubricant would not be viewed as benefitting the process.

Moreover, with respect to preparing a final dosage form containing a solubility enhanced form of an API, specifically in the form of an amorphous solid dispersion, conventional wisdom suggests that the use of lubricants in the outer phase of the dosage form, *i.e.*, external to the amorphous solid dispersion phase, can negatively impact dissolution because lubricants tend to be insoluble crystalline materials that can act as sites for nucleation and crystal growth for poorly water soluble drugs that are supersaturated in aqueous media. Thus, it is counter-intuitive to include a lubricant in the amorphous solid dispersion phase of a formulated API.

### SUMMARY

Thus, in accordance with the present disclosure, there is provided a method of making a pharmaceutical composition comprising (a) providing an active pharmaceutical ingredient (API), or a pharmaceutically acceptable salt, ester, derivative, analog, prodrug or solvate thereof, and one or more pharmaceutically acceptable excipients including a non-polymeric lubricant; (b) processing the materials of step (a) using thermal processing or solvent evaporation, wherein the processing of the API and the one or more pharmaceutically acceptable excipients forms an amorphous pharmaceutical composite. The resulting composition thus contains the non-polymeric lubricant in an amorphous solid dispersion phase, and it exists there in an amorphous state. In another aspect, the non-polymeric lubricant and the drug are supersaturated in the aqueous media, leading to stabilizing solution interactions. The non-polymeric lubricant may be poorly soluble in water, or water insoluble, and/or or may be crystalline in its pre-compounding state. The thermal processing may be hot melt extrusion or thermokinetic processing.

The pharmaceutical composition may comprise a more than one active pharmaceutical ingredients. The one or more pharmaceutically acceptable excipient may comprise a surfactant and/or a pharmaceutical polymer, including one or more surfactants and one or more polymer carriers. Step (b) may be performed at a maximum temperature of about 250 °C, about 225 °C, about 200 °C, about 180 °C, about 150 °C, about 150 °C to 250 °C, or about 180 °C to 250 °C. In a particular embodiment, the API specifically does not include vemurafenib.

The non-polymeric lubricant may comprise magnesium stearate, glyceryl behenate, calcium stearate, (sodium) stearyl fumarate, glyceryl monostearate, glyceryl palmitostearate, myristic acid, palmitic acid, stearic acid, or zinc stearate.

The pharmaceutically acceptable excipients may further comprise an agent selected from the group consisting of poly(vinyl acetate)-co-poly(vinylpyrrolidone) copolymer, ethylcellulose, hydroxypropylcellulose, cellulose acetate butyrate, poly(vinylpyrrolidone), poly(ethylene glycol), poly(ethylene oxide), poly(vinyl alcohol), hydroxypropyl methylcellulose, ethylcellulose, hydroxyethylcellulose, sodium carboxymethyl-cellulose, dimethylaminoethyl methacrylate-methacrylic acid ester copolymer, ethylacrylate-methylmethacrylate copolymer, cellulose acetate phthalate, cellulose acetate trimelletate, poly(vinyl acetate) phthalate, hydroxypropylmethylcellulose phthalate, poly(methacrylate ethylacrylate) (1:1) copolymer, poly(methacrylate methylmethacrylate) (1:1) copolymer, poly(methacrylate methylmethacrylate) (1:2) copolymer, hydroxypropylmethylcellulose acetate succinate and polyvinyl caprolactam-polyvinyl acetate-polyethylene glycol graft copolymer sodium dodecyl sulfate, dioctyl sodium sulphosuccinate, polyoxyethylene (20) sorbitan monooleate, glycerol polyethylene glycol oxystearate-fatty acid glycerol polyglycol esters-polyethylene glycols-glycerol ethoxylate, glycerol-polyethylene glycol ricinoleate-fatty acid esters of polyethyleneglycol-polyethylene glycols-ethoxylated glycerol, vitamin E TPGS and sorbitan laurate.

The pharmaceutical polymer may comprise an agent selected from the group consisting of poly(vinyl acetate)-co-poly(vinylpyrrolidone) copolymer, ethylcellulose, hydroxypropylcellulose, cellulose acetate butyrate, poly(vinylpyrrolidone), poly(ethylene glycol), poly(ethylene oxide), poly(vinyl alcohol), hydroxypropyl methylcellulose, ethylcellulose, hydroxyethylcellulose, sodium carboxymethyl-cellulose, dimethylaminoethyl methacrylate-methacrylic acid ester copolymer, ethylacrylate-methylmethacrylate copolymer, cellulose acetate phthalate, cellulose acetate trimelletate, poly(vinyl acetate) phthalate, hydroxypropylmethylcellulose phthalate, poly(methacrylate ethylacrylate) (1:1) copolymer, poly(methacrylate methylmethacrylate) (1:1) copolymer, poly(methacrylate methylmethacrylate) (1:2) copolymer, hydroxypropylmethylcellulose acetate succinate and polyvinyl caprolactam-polyvinyl acetate-polyethylene glycol graft copolymer.

The surfactant may comprise an agent selected from the group consisting of sodium dodecyl sulfate, dioctyl sodium sulphosuccinate, polyoxyethylene (20) sorbitan monooleate, glycerol polyethylene glycol oxystearate-fatty acid glycerol polyglycol esters-polyethylene glycols-glycerol ethoxylate, glycerol-polyethylene glycol ricinoleate-fatty acid esters of polyethyleneglycol-polyethylene glycols-ethoxylated glycerol, vitamin E TPGS, and sorbitan laurate, and the pharmaceutical polymer comprises an agent selected from a group consisting of poly(vinylpyrrolidone), ethylacrylate-methylmethacrylate copolymer, poly(methacrylate ethylacrylate) (1:1) copolymer, hydroxypropylmethylcellulose acetate succinate, poly(butyl methacylate-co-(2-dimethylaminoethyl) methacrylate-co-methyl methacrylate) 1:2:1 and polyvinyl caprolactam-polyvinyl acetate-polyethylene glycol graft copolymer.

The one or more pharmaceutically acceptable excipients may comprise a processing agent, such as a plasticizer.

The one or more pharmaceutically acceptable excipients may comprise a pharmaceutical polymer of high melt viscosity and/or a thermally labile pharmaceutical polymer.

In another embodiment, there is provided a pharmaceutical composition comprising an amorphous dispersion of an active pharmaceutical ingredient, or a pharmaceutically acceptable salt, ester, derivative, analog, prodrug or solvate thereof, and one or more pharmaceutically acceptable excipients, wherein the one or more pharmaceutically acceptable excipients comprises a non-polymeric lubricant that is co-processed with the API. The composition thus contains the non-polymeric lubricant in an amorphous solid dispersion phase, and it exists there in an amorphous state. The pharmaceutical may comprise more than one active pharmaceutical ingredient. In a particular embodiment, the API specifically does not include vemurafenib. The non-polymeric lubricant may be poorly soluble in water, or water insoluble, and/or or may be crystalline in its pre-compounding state.

The non-polymeric lubricant may comprise magnesium stearate, glyceryl behenate, calcium stearate, (sodium) stearyl fumarate, glyceryl monostearate, glyceryl palmitostearate, myristic acid, palmitic acid, stearic acid, or zinc stearate.

The one or more pharmaceutically acceptable excipient may comprise a surfactant, a processing agent, or a plasticizer.

The pharmaceutically acceptable excipients may further comprise an agent selected from the group consisting of poly(vinyl acetate)-co-poly(vinylpyrrolidone) copolymer, ethylcellulose, hydroxypropylcellulose, cellulose acetate butyrate, poly(vinylpyrrolidone), poly(ethylene glycol), poly(ethylene oxide), poly(vinyl alcohol), hydroxypropyl methylcellulose, ethylcellulose, hydroxyethylcellulose, sodium carboxymethyl-cellulose, dimethylaminoethyl methacrylate-methacrylic acid ester copolymer, ethylacrylate-methylmethacrylate copolymer, cellulose acetate phthalate, cellulose acetate trimelletate, poly(vinyl acetate) phthalate, hydroxypropylmethylcellulose phthalate, poly(methacrylate ethylacrylate) (1:1) copolymer, poly(methacrylate methylmethacrylate) (1:1) copolymer, poly(methacrylate methylmethacrylate) (1:2) copolymer, hydroxypropylmethylcellulose acetate succinate and polyvinyl caprolactam-polyvinyl acetate-polyethylene glycol graft copolymer sodium dodecyl sulfate, dioctyl sodium sulphosuccinate, polyoxyethylene (20) sorbitan monooleate, glycerol polyethylene glycol oxystearate-fatty acid glycerol polyglycol esters-polyethylene glycols-glycerol ethoxylate, glycerol-polyethylene glycol ricinoleate-fatty acid esters of polyethyleneglycol-polyethylene glycols-ethoxylated glycerol, vitamin E TPGS and sorbitan laurate.

The pharmaceutical polymer may comprise an agent selected from the group consisting of poly(vinyl acetate)-co-poly(vinylpyrrolidone) copolymer, ethylcellulose, hydroxypropylcellulose, cellulose acetate butyrate, poly(vinylpyrrolidone), poly(ethylene glycol), poly(ethylene oxide), poly(vinyl alcohol), hydroxypropyl methylcellulose, ethylcellulose, hydroxyethylcellulose, sodium carboxymethyl-cellulose, dimethylaminoethyl methacrylate-methacrylic acid ester copolymer, ethylacrylate-methylmethacrylate copolymer, cellulose acetate phthalate, cellulose acetate trimelletate, poly(vinyl acetate) phthalate, hydroxypropylmethylcellulose phthalate, poly(methacrylate ethylacrylate) (1:1) copolymer, poly(methacrylate methylmethacrylate) (1:1) copolymer, poly(methacrylate methylmethacrylate) (1:2) copolymer, hydroxypropylmethylcellulose acetate succinate and polyvinyl caprolactam-polyvinyl acetate-polyethylene glycol graft copolymer.

The surfactant may comprise an agent selected from the group consisting of sodium dodecyl sulfate, dioctyl sodium sulphosuccinate, polyoxyethylene (20) sorbitan monooleate, glycerol polyethylene glycol oxystearate-fatty acid glycerol polyglycol esters-polyethylene glycols-glycerol ethoxylate, glycerol-polyethylene glycol ricinoleate-fatty acid esters of polyethyleneglycol-polyethylene glycols-ethoxylated glycerol, vitamin E TPGS, and sorbitan laurate, and the pharmaceutical polymer comprises an agent selected from a group consisting of poly(vinylpyrrolidone), hydroxypropylcellulose, poly(vinyl alcohol), hydroxypropyl methylcellulose, hydroxyethylcellulose, and sodium carboxymethyl-cellulose.and polyvinyl caprolactam-polyvinyl acetate-polyethylene glycol graft copolymer.

The pharmaceutically acceptable excipients may further comprise an agent selected from the group consisting of sodium dodecyl sulfate, dioctyl sodium sulphosuccinate, polyoxyethylene (20) sorbitan monooleate, glycerol polyethylene glycol oxystearate-fatty acid glycerol polyglycol esters-polyethylene glycols-glycerol ethoxylate, glycerol-polyethylene glycol ricinoleate-fatty acid esters of polyethyleneglycol-polyethylene glycols-ethoxylated glycerol, vitamin E TPGS, sorbitan laurate, poly(vinyl acetate)-co-poly(vinylpyrrolidone) copolymer, hydroxypropylcellulose, poly(vinylpyrrolidone), poly(ethylene glycol), poly(ethylene oxide), poly(vinyl alcohol), hydroxypropyl methylcellulose, ethylcellulose, hydroxyethylcellulose, sodium carboxymethyl-cellulose, and polyvinyl caprolactam-polyvinyl acetate-polyethylene glycol graft copolymer.

The pharmaceutical composition may not contain a processing agent, and/or may not contain a plasticizer. The composition may be a composite and is a homogenous, heterogeneous, or heterogeneously homogenous composition.

The one or more pharmaceutically acceptable excipients may further comprise a pharmaceutical polymer of high melt viscosity, and or a thermally labile pharmaceutical polymer.

The peak solubility of the active pharmaceutical ingredient and the reference standard the active pharmaceutical ingredient in an aqueous buffer with a pH range of 4 to 8 have a ratio of greater than 3:1, greater than 10:1, greater than 20:1 or greater than 30:1. The Cₘₐₓ of the active pharmaceutical ingredient in the composition and Cₘₐₓ of the reference standard the active pharmaceutical ingredient have a ratio that is greater than 6:1.

The pharmaceutical composition may be formulated into an oral dosage form, such as a tablet, a capsule, or a sachet.

In yet a further embodiment, there is provided a pharmaceutical composition produced by a process comprising the steps of (a) providing an active pharmaceutical ingredient and one or more pharmaceutically acceptable excipients including a non-polymeric lubricant; (b) processing the materials of step (a) using thermal processing or solvent evaporation, wherein the processing of the active pharmaceutical ingredient and the one or more pharmaceutically acceptable excipients forms an amorphous pharmaceutical composition. The composition thus contains the non-polymeric lubricant in an amorphous solid dispersion phase, and it exists there in an amorphous state. The thermal processing may be hot melt extrusion or thermokinetic processing. The non-polymeric lubricant may be poorly soluble in water, or water insoluble, and/or or may be crystalline in its pre-compounding state.

The one or more pharmaceutically acceptable excipients may further include a non-ionic pharmaceutical polymer, an ionic pharmaceutical polymer, a water soluble pharmaceutical polymer, cellulosic pharmaceutical polymer, a non-ionic, water soluble pharmaceutical polymer, a non-ionic, cellulosic pharmaceutical polymer, a water soluble, cellulosic pharmaceutical polymer, a thermally labile pharmaceutical polymer, a high melt viscosity pharmaceutical polymer, and/or a cross-linked pharmaceutical polymer. In a particular embodiment, the API specifically does not include vemurafenib.

The non-polymeric lubricant may comprise magnesium stearate, glyceryl behenate, calcium stearate, (sodium) stearyl fumarate, glyceryl monostearate, glyceryl palmitostearate, myristic acid, palmitic acid, stearic acid, or zinc stearate.

The pharmaceutical composition may comprise a processing agent, such as a plasticizer. The pharmaceutical composition may further comprise one or more active pharmaceutical ingredient(s). The pharmaceutical composition may be combined with a co-processed with one or more active pharmaceutical ingredient(s) in a final dosage form. The pharmaceutical composition may be admixed with one or more active pharmaceutical ingredient(s) in a final dosage form.

The thermokinetic processing may be conducted in a thermokinetic chamber. A thermokinetic chamber is an enclosed vessel or chamber in which TKC occurs. In one aspect, the average temperature inside the chamber is ramped up to a pre-defined final temperature over the duration of processing to achieve optimal thermokinetic mixing of the active pharmaceutical ingredient and the one or more pharmaceutically acceptable excipients, adjuvants, additional APIs, or any combination thereof, into a composite. In another aspect, multiple speeds are used during a single, rotationally continuous TKC operation to achieve optimal thermokinetic mixing of the active pharmaceutical ingredient and one or more pharmaceutically acceptable excipients, adjuvants, additional APIs, or any combination thereof, into a composite with minimal thermal degradation. The length of processing and exposure to elevated temperatures or speeds during thermokinetic mixing will generally be below the thermal sensitivity threshold of the active pharmaceutical ingredient, excipient(s), adjuvant(s), or additional API(s). In another aspect, the thermokinetic processing is performed at an average temperature at or below the melting point of the active pharmaceutical ingredient, excipient(s), adjuvant(s), or additional API(s); the thermokinetic processing is performed at an average temperature at or below the glass transition temperature of the active pharmaceutical ingredient, excipient(s), adjuvant(s), or additional API(s); or the thermokinetic processing is performed at an average temperature at or below the molten transition point of the active pharmaceutical ingredient, excipient(s), adjuvant(s), or additional API(s).

In one aspect, the active pharmaceutical ingredient composite made by thermal processing or solvent evaporation is a homogenous, heterogeneous, or heterogeneously homogenous composite or an amorphous composite. In another aspect, the method, the active pharmaceutical ingredient compositions and composite of the present disclosure may be adapted for oral or non-oral administration, for example buccal, sublingual, intravenous, parenteral, pulmonary, rectal, vaginal, topical, urethral, otic, ocular, or transdermal administration. In another aspect, the non-polymeric lubricant and the drug are supersaturated in the aqueous media, leading to stabilizing solution interactions.

In another aspect, the thermal processing may be conducted with or without a processing agent. Examples of processing agents include a plasticizer, a thermal lubricant, an organic solvent, an agent that facilitates melt blending, and an agent that facilitates downstream processing (*e.g.*, lecithin). The composite may also include a carrier, *e.g.*, a polymer with a high melt viscosity. In another aspect, the release rate profile of the active pharmaceutical ingredient is determined by the one or more excipients of the composition. As such, the composition may be formulated for immediate release, mixed release, extended release or combinations thereof. In another aspect, the particle size of the active pharmaceutical ingredient is reduced in an excipient/carrier system in which the active pharmaceutical ingredient is not miscible, not compatible, or not miscible or compatible. In one aspect, the active pharmaceutical ingredient is formulated as a nanocomposite with an excipient, a carrier, an adjuvant, or any combination thereof. In a particular embodiment, the API specifically does not include vemurafenib.

The non-polymeric lubricant may be poorly soluble in water, or water insoluble, and/or or may be crystalline in its pre-compounding state. The non-polymeric lubricant may comprise magnesium stearate, glyceryl behenate, calcium stearate, (sodium) stearyl fumarate, glyceryl monostearate, glyceryl palmitostearate, myristic acid, palmitic acid, stearic acid, or zinc stearate.

In certain embodiments, the thermokinetic processing substantially eliminates the active pharmaceutical ingredient, excipient, adjuvant or additional API degradation. For example, TKC may generate compositions and composites with less than about 2.0%, 1.0%, 0.75%, 0.5%, 0.1%, 0.05%, or 0.01% degradation products of the active pharmaceutical ingredient, adjuvant, excipient or additional API. This advantage is important for the active pharmaceutical ingredient, which is subject to recrystallization during washing and drying during the MBP process. In other embodiments, TKC may generate compositions with a minimum of at least about 90%, 91%, 92%, 93%, 94%, 95%, 96%, 97%, 98%, 99%, or 99.9% drug potency with respect to the active pharmaceutical ingredient. Examples of TKC may be performed for less than 5, 10, 15, 20, 25, 30, 35, 40, 45, 50, 55, 60, 75, 100, 120, 150, 180, 240 and 300 seconds. Generally, TKC may be performed for less than 5, 10, 15, 20, 25, 30, 35, 40, 45, 50, 55, 60, 75, 100, 120, 150, 180, 240 and 300 seconds, and any ranges therein. In certain embodiments, the active pharmaceutical ingredient has amorphous, crystalline, or intermediate morphology.

In certain embodiments, the formulations may provide for enhanced solubility of the active pharmaceutical ingredient through the mixing of the active pharmaceutical ingredient with pharmaceutically acceptable polymers, carriers, surfactants, excipients, adjuvants or any combination thereof. Thus, for example, compositions which display enhanced solubility are comprised of the active pharmaceutical ingredient and a surfactant or surfactants, the active pharmaceutical ingredient and a pharmaceutical carrier (thermal binder) or carriers, or the active pharmaceutical ingredient and a combination of a surfactant and pharmaceutical carrier or surfactants and carriers. In a particular embodiment, the API specifically does not include vemurafenib.

A further embodiment of the present disclosure is a pharmaceutical composition comprising the active pharmaceutical ingredient, and one or more pharmaceutically acceptable excipients including a non-polymeric lubricant, adjuvants, additional APIs, or a combination thereof, wherein a peak solubility of the active pharmaceutical ingredient in the composition is greater than about 6 µg/mL, about 7 µg/mL, about 8 µg/mL, about 9 µg/mL, about 10 µg/mL, about 11 µg/mL, about 12 µg/mL, about 13 µg/mL, about 14 µg/mL, about 15 µg/mL, about 16 µg/mL, about 20 µg/mL, about 25 µg/mL, about 30 µg/mL, about 35 µg/mL, about 40 µg/mL, 45 µg/mL, about 50 µg/mL or about 60 µg/mL in an aqueous buffer of pH between 4 and 8. In a particular embodiment, the API specifically does not include vemurafenib. The non-polymeric lubricant may be poorly soluble in water, or water insoluble, and/or or may be crystalline in its pre-compounding state.

A further embodiment of the present disclosure is a pharmaceutical composition comprising the active pharmaceutical ingredient and one or more pharmaceutically acceptable excipients including a non-polymeric lubricant, adjuvants, additional APIs, or a combination thereof, wherein a ratio of peak solubility of the active pharmaceutical ingredient in the composition over peak solubility of a reference standard of the active pharmaceutical ingredient is greater than about 3:1, about 4:1, about 5:1, about 6:1, about 7:1, about 8:1, about 9:1, or about 10:1. In a particular embodiment, the API specifically does not include vemurafenib. The non-polymeric lubricant may be poorly soluble in water, or water insoluble, and/or or may be crystalline in its pre-compounding state.

A further embodiment of the present disclosure is a method of formulating a pharmaceutical composition comprising the active pharmaceutical ingredient and one or more pharmaceutically acceptable excipients including a non-polymeric lubricant, adjuvants, additional APIs, or any combination thereof, by TKC to increase bioavailability of the active pharmaceutical ingredient, comprising thermokinetic processing of the active pharmaceutical ingredient with the one or more pharmaceutically acceptable excipients, adjuvants, additional APIs, or any combination thereof until melt blended into a composite. In a particular embodiment, the API specifically does not include vemurafenib. The non-polymeric lubricant may be poorly soluble in water, or water insoluble, and/or or may be crystalline in its pre-compounding state.

A further embodiment of the present disclosure is a pharmaceutical composition comprising the active pharmaceutical ingredient and one or more pharmaceutically acceptable excipients including a non-polymeric lubricant, adjuvants, additional APIs, or any combination thereof, processed into a composite, wherein the composite is a homogenous, heterogeneous, or heterogeneously homogenous composition which has a less than about 1.0%, about 2%, about 3%, about 4% or about 5%, about 6%, about 7%, about 8%, about 9%, or about 10% degradation products of the active pharmaceutical ingredient. In a particular embodiment, the API specifically does not include vemurafenib. The non-polymeric lubricant may be poorly soluble in water, or water insoluble, and/or or may be crystalline in its pre-compounding state.

Although making and using various embodiments of the present disclosure are discussed above and in detail below, it should be appreciated that the present disclosure provides many inventive concepts that may be embodied in a wide variety of contexts. The specific aspects and embodiments discussed herein are merely illustrative of ways to make and use the disclosure, and do not limit the scope of the disclosure.

### BRIEF DESCRIPTION OF THE DRAWINGS

The following drawings form part of the present specification and are included to further demonstrate certain aspects of the present disclosure. The disclosure may be better understood by reference to one or more of these drawings in combination with the detailed description of specific embodiments presented herein.

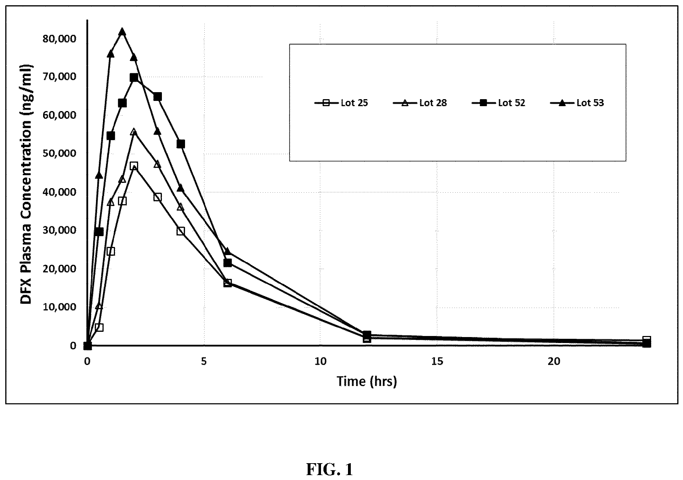

**FIG. 1****.** Amorphous dispersions of DFX using two polymer carrier systems were produced, including those with and without internal MgSt. Tablets containing those dispersions were prepared and dosed to beagle dogs at a dose of 36 mg/kg. The AUCs were roughly 50% higher for the tablets containing ASDs with internal MgSt (solid symbols) *versus* without (open symbols).

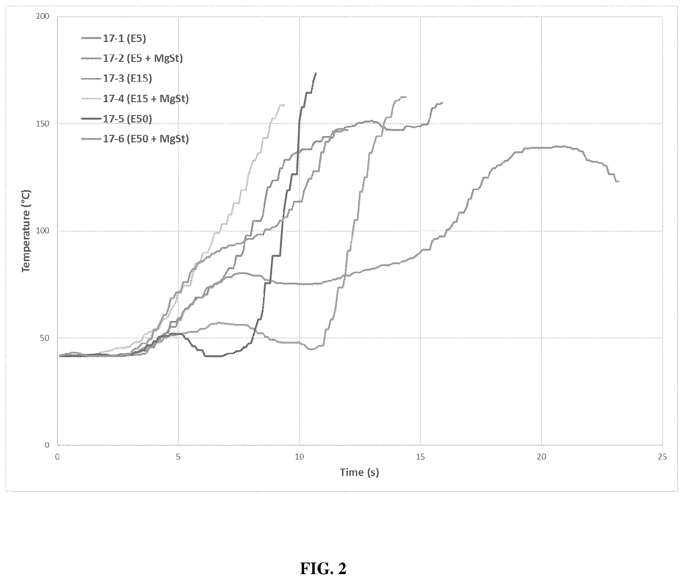

**FIG. 2****.** Thermokinetic Compounding of Variable Hypromellose Compositions. The maximum temperature was below the melting point of itraconazole with temperature elevation for less than 20 seconds. All profiles permitted thermal processing to render the itraconazole/pharmaceutical polymer/lubricant (where applicable) compositions amorphous.

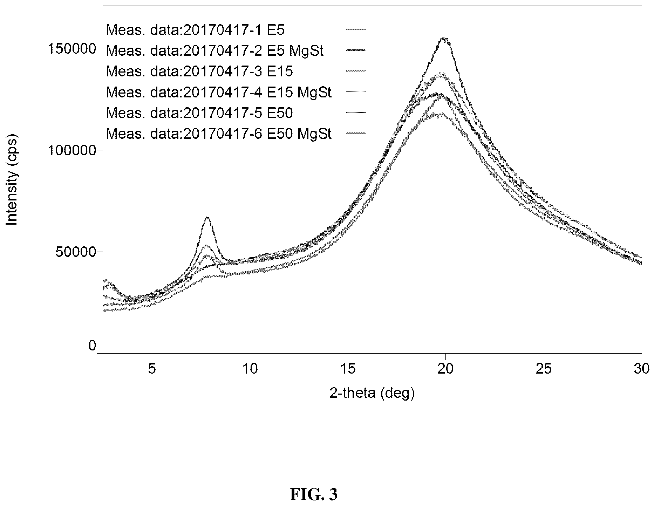

**FIG. 3****.** X-ray Powder Diffraction of Variable Hypromellose Compositions. The results demonstrated that the thermokinetic compounding batches were amorphous for all itraconazole/pharmaceutical polymer/lubricant (where applicable) compositions.

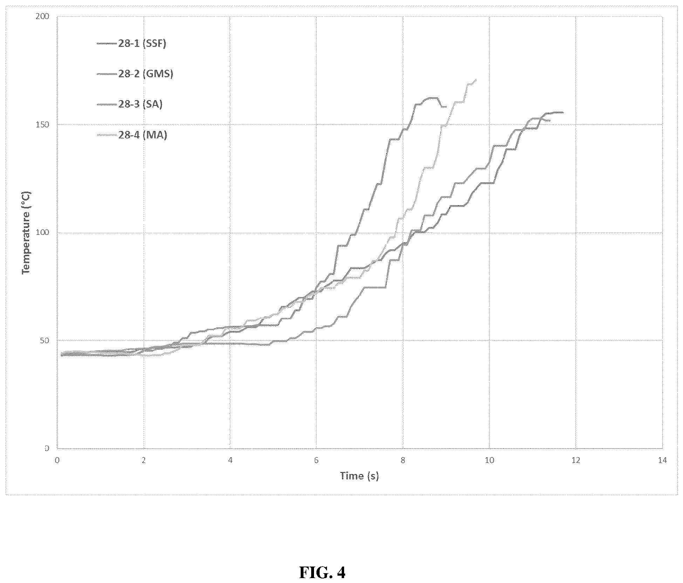

**FIG. 4****.** Thermokinetic Compounding of Variable Lubricant Compositions. The maximum temperature was below the melting point of itraconazole with temperature elevation for less than 10 seconds. All profiles permitted thermal processing to render the itraconazole/pharmaceutical polymer/lubricant compositions amorphous.

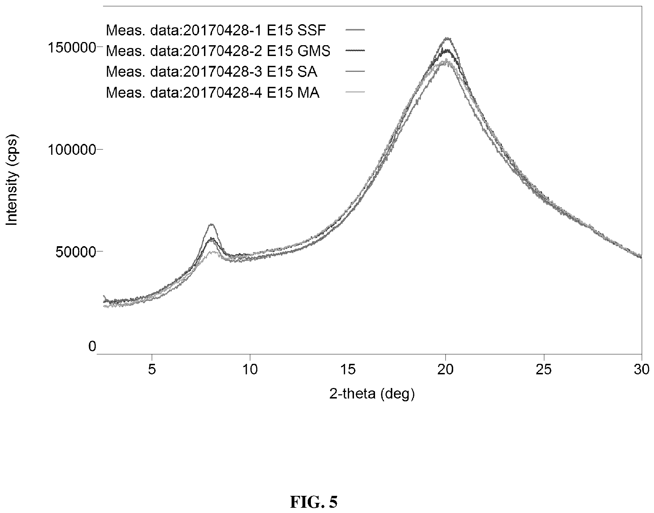

**FIG. 5****.** X-ray Powder Diffraction of Variable Lubricant Compositions. The results demonstrated that the thermokinetic compounding batches were amorphous for all itraconazole/pharmaceutical polymer/lubricant (where applicable) compositions.

### DETAILED DESCRIPTION

Considering the issues associated with lubricants external to the amorphous dispersion phase of the tablet, as discussed above, a formulation scientist would be highly disinclined to include lubricants within the amorphous solid dispersion phase where the non-polymeric lubricant material would be more intimately associated with the drug molecule and would be expected to have an even greater negative impact on performance of the dosage form with respect to solubility, dissolution rate, and bioavailability. Furthermore, for the conventional processes of making amorphous solid dispersions (spray drying and melt extrusion), there is no inherent processing advantage of including conventional pharmaceutical crystalline powder lubricants in the formulation as there is no essential powder flow component to these processes.

However, the inventors' research has shown that conventional pharmaceutical crystalline powder lubricants when rendered amorphous within the internal phase of an amorphous solid dispersion can substantially improve solubility, dissolution, and bioavailability of the formulation. Specifically, it is believed that, when rendered amorphous in the solid dispersion system, the non-polymeric lubricant molecules are able to dissolve into (supersaturate) aqueous media along with the drug and then act as a stabilizing agent against drug nucleation and/or crystal growth thus increasing the extent and duration of drug supersaturation in aqueous media. This aqueous drug concentration enhancing effect thus leads to increased bioavailability upon oral administration by increasing the concentration of free drug molecules available for absorption in gastro-intestinal fluids. Indeed, this approach may be more potent than other approaches as the effect was observed with a minimal concentration of the non-polymeric lubricant, as little as 0.5% w/w. This may allow for performance enhancement above and beyond what is possible by other approaches.

The rendering of the conventional pharmaceutical crystalline powder lubricants amorphous in the solid dispersion is an important feature since, in a crystalline form, the non-polymeric lubricant material would promote nucleation and crystal growth of the drug in aqueous media because the non-polymeric lubricant would not enter aqueous solution, and thus it would act as a surface for nucleation and crystal growth of the drug. Alternatively, when rendered amorphous in the solid dispersion, the non-polymeric lubricant is able to supersaturate the aqueous media with the drug, thus allowing for intermolecular interactions in aqueous media between the drug and lubricant that stabilize the drug against precipitating from solution.

The proposed mechanism of solution stabilization of supersaturated aqueous solutions with poorly water soluble drug molecules by conventional lubricants is identical to the literature descriptions of the stabilizing mechanism by traditional surfactants. However, the inventors' research also shows the mechanism of solution stabilization of a drug by a lubricant molecule is more efficient than that of a traditional surfactant. Indeed, they have observed substantial concentration enhancement with lubricants levels as low as 0.5%, and also observed marked increases in aqueous drug concentrations with the addition of lubricants to amorphous solid dispersions already containing a substantial concentration (>5% w/w) of a conventional surfactant.

The discovery of the concentration enhancing effects of conventional pharmaceutical crystalline powder lubricants on poorly water soluble drugs from amorphous solid dispersion formulations was realized when developing such formulations using thermokinetic compounding (TKC). Unlike spray drying and melt extrusion, the inclusion of a conventional pharmaceutical crystalline powder lubricants has inherent processing advantages with TKC as there is a powder flow component to the initial stage of the process and the incorporation of the non-polymeric lubricant mitigates powder adhesion to the processing chamber and thus enhances product yield and uniformity. Therefore, conventional pharmaceutical crystalline powder lubricants are commonly incorporated into TKC formulations to improve processing efficiency and product quality. The dissolution and bioavailability enhancing effects of incorporating lubricants into amorphous solid dispersion (ASD) formulations was surprisingly observed when comparing *in vitro* and *in vivo* performance of drug-polymer ASD formulations with and without a lubricant and realizing a substantial performance enhancing effect with the inclusion of lubricant at concentrations as low as 0.5% (w/w) in the formulation. Even more surprisingly, performance enhancing effect was also observed for drug-polymer-surfactant formulations by comparing in-vitro and/or in-vivo performance of such formulations with and without a lubricant. The marked performance enhancement in this case was especially surprising because it was expected that the stabilizing effect of the traditional surfactant would supersede that of the non-polymeric lubricant; however, what was observed was even greater stabilizing of supersaturation effect with the inclusion of the non-polymeric lubricant.

While this discovery was made during ASD development for numerous poorly water soluble drugs with TKC, the inherent processing advantages of incorporating conventional pharmaceutical crystalline powder lubricants in TKC would not be associated with other processes. As such, the compositions described here are not limited to those made using TKC processing. In fact, these disclosed compositions can be created using melt extrusion, and potentially spray drying, given a common organic solvent for the drug and all excipient components including the non-polymeric lubricant. Therefore, the current disclosure provides new pharmaceutical compositions comprising at least one API, at least one excipient carrier, and at least one conventional pharmaceutical lubricant that is poorly water soluble and crystalline in its bulk form, wherein the drug and the non-polymeric lubricant are substantially amorphous. This composition can be achieved by co-processing the above components by thermal and solvent processing methods, e.g., TKC, HME, and spray drying, for example.

Applicants thus describe improved active pharmaceutical ingredient compositions and methods for their manufacture. These methods permit thermal processing to produce an amorphous solid dispersion of the active pharmaceutical ingredient with high amorphous drug loading. In particular, they include a composition that includes at least one active pharmaceutical ingredient and a crystalline, non-polymeric, poorly soluble lubricant. After processing, both the active pharmaceutical ingredient and the lubricant are amorphous in the composition. While exemplified, the processing is not necessarily limited to thermokinetic mixing. These and other aspects of the disclosure are discussed in detail below.

### I. Definitions

To facilitate the understanding of this disclosure, a number of terms are defined below. Terms defined herein have meanings as commonly understood by a person of ordinary skill in the areas relevant to the present disclosure. Terms such as "a", "an" and "the" are not intended to refer to only a singular entity, but include the general class of which a specific example may be used for illustration.

With regard to the values or ranges recited herein, the term "about" is intended to capture variations above and below the stated number that may achieve substantially the same results as the stated number. In the present disclosure, each of the variously stated ranges is intended to be continuous so as to include each numerical parameter between the stated minimum and maximum value of each range. For example, a range of about 1 to about 4 includes about 1, 1, about 2, 2, about 3, 3, about 4, and 4. The terminology herein is used to describe specific embodiments of the disclosure, but their usage does not delimit the disclosure, except as outlined in the claims.

All publications and patent applications mentioned in the specification are indicative of the level of skill of those skilled in the art to which this disclosure pertains. All publications and patent applications are herein incorporated by reference to the same extent as if each individual publication or patent application was specifically and individually indicated to be incorporated by reference.

The use of the word "a" or "an" when used in conjunction with the term "comprising" in the claims and/or the specification may mean "one," but it is also consistent with the meaning of "one or more," "at least one," and "one or more than one." The use of the term "or" in the claims is used to mean "and/or" unless explicitly indicated to refer to alternatives only or the alternatives are mutually exclusive, although the disclosure supports a definition that refers to only alternatives and "and/or." Throughout this application, the term "about" is used to indicate that a value includes the inherent variation of error for the device, the method being employed to determine the value, or the variation that exists among the study subjects.

As used in this specification and claims, the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

The term "or combinations thereof" as used herein refers to all permutations and combinations of the listed items preceding the term. For example, "A, B, C, or combinations thereof" is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, MB, BBC, AAABCCCC, CBBAAA, CABABB, and so forth. The skilled artisan will understand that typically there is no limit on the number of items or terms in any combination, unless otherwise apparent from the context.

As used herein, the term "thermokinetic compounding" or "TKC" refers to a method of thermokinetic mixing until melt blended. TKC may also be described as a thermokinetic mixing process or thermokinetic processing in which processing ends at a point sometime prior to agglomeration. The commercial name for this process is "KinetiSol^{®}".

As used herein, the phrase "a homogenous, heterogenous, or heterogeneously homogenous composite or an amorphous composite" refers to the various compositions that can be made using the TKC method.

As used herein, the term "heterogeneously homogenous composite" refers to a material composition having at least two different materials that are evenly and uniformly distributed throughout the volume.

As used herein, the phrase "reference standard active pharmaceutical ingredient" means the most thermodynamically stable form of the active pharmaceutical ingredient that is currently available.

As used herein, the term "substantial degradation," in conjunction with the term "the active pharmaceutical ingredient" or "additional API(s)" refers to degradation leading to the generation of impurities at levels beyond the threshold that has been qualified by toxicology studies, or beyond the allowable threshold for unknown impurities. *See*, *for example* Guidance for Industry, Q3B(R2) Impurities in New Drug Products (International Committee for Harmonization, published by the U.S. Department of Health and Human Services, Food and Drug Administration, Center for Drug Evaluation and Research (CDER), Center for Biologics Evaluation and Research, July, 2006. As used herein, the term "substantial degradation," in conjunction with the term "excipient" refers to decomposition of the excipient to the extent that the excipient would no longer meet the specifications set forth in an official monograph of an accepted pharmacopeia, *e*.*g*., the United States Pharmacopeia.

As used herein, the term "high melt viscosity" refers to melt viscosities greater than 10,000 Pa*s.

As used herein, the term "thermally labile API" refers to an API that degrades at its crystalline melting point, or one that degrades at temperatures below the crystalline melting point when in a non-crystalline (amorphous) form. As used herein, the term "thermolabile polymer" refers to a polymer that degrades at or below about 200°C.

Whether the composition of the present disclosure is a homogenous, heterogenous, or heterogeneously homogenous composition, an amorphous composition or combinations thereof, the TKC processing conditions can produce a composition with a glass transition temperature that is higher than the glass transition temperature of an identical combination of the drug and pharmaceutically acceptable excipients, adjuvants, additional APIs, or any combination thereof, thermally processed or processed using the MBP method, for example either with or without the use of a plasticizer. The TKC processing conditions can also produce a composition with a single glass transition temperature, wherein an identical combination of the identical API and pharmaceutically acceptable excipients, adjuvants, additional APIs, or any combination thereof, processed thermally or processed using the MBP method, has two or more glass transition temperatures. In other embodiments, the pharmaceutical compositions of the present disclosure have a single glass transition temperature that is at least about 20%, 30%, 40%, 50%, 60%, 70%, 80%, or 90% higher than the lowest glass transition temperature of the identical combination processed thermally or processed using the MBP method. Alternatively, the compositions made using thermokinetic processing may generate compositions with a minimum of at least about 90%, 91%, 92%, 93%, 94%, 95%, 96%, 97%, 98%, 99%, or 99.9% therapeutic potency with respect to each drug.

As used herein, the term "significantly higher" in conjunction with glass transition temperatures, refers to compositions that have a glass transition temperature that is at least about 20% higher than the lowest glass transition temperature of the identical formulation thermally processed or processed using the MBP method.

As used herein, the term "thermokinetic chamber" refers to an enclosed vessel or chamber in which the TKC method is used to make the novel compositions of the present disclosure.

As used herein, "thermally processed" or "processed thermally" means that components are processed by hot melt extrusion, melt granulation, compression molding, tablet compression, capsule filling, film-coating, or injection molding.

As used herein, "extrusion" is the well-known method of applying pressure to a damp or melted composition until it flows through an orifice or a defined opening. The extrudable length varies with the physical characteristics of the material to be extruded, the method of extrusion, and the process of manipulation of the particles after extrusion. Various types of extrusion devices can be employed, such as screw, sieve and basket, roll, and ram extruders. Furthermore, the extrusion can be carried out through melt extrusion. Components of the present disclosure can be melted and extruded with a continuous, solvent free extrusion process, with or without inclusion of additives. Such processes are well-known to skilled practitioners in the art.

As used herein, "spray congealing" is a method that is generally used in changing the structure of materials, to obtain free flowing powders from liquids and to provide pellets. Spray congealing is a process in which a substance of interest is allowed to melt, disperse, or dissolve in a hot melt of other additives, and is then sprayed into an air chamber wherein the temperature is below the melting point of the formulation components, to provide congealed pellets. Such a process is well-known to skilled practitioners in the art.

As used herein, "solvent dehydration" or "spray drying technique" is commonly employed to produce a dry powder from a liquid or slurry by rapidly drying with a hot gas. This is one preferred method of drying many thermally-sensitive materials such as foods and pharmaceuticals. Water or organic solvent based formulations can be spray dried by using inert process gas, such as nitrogen, argon and the like. Such a process is well-known to skilled practitioners in the art.

In certain embodiments, the pharmaceutical formulations of the present disclosure can be processed by the techniques of extrusion, melt extrusion, spray congealing, spray drying or any other conventional technique to provide solid compositions from solution, emulsions suspensions or other mixtures of solids and liquids or liquids and liquids.

As used herein, "bioavailability" is a term meaning the degree to which a drug becomes available to the target tissue after being administered to the body. Poor bioavailability is a significant problem encountered in the development of pharmaceutical compositions, particularly those containing a drug that is not highly soluble. In certain embodiments such as formulations of proteins, the proteins may be water soluble, poorly soluble, not highly soluble, or not soluble. The skilled artisan will recognize that various methodologies may be used to increase the solubility of proteins, e.g., use of different solvents, excipients, carriers, formation of fusion proteins, targeted manipulation of the amino acid sequence, glycosylation, lipidation, degradation, combination with one or more salts and the addition of various salts.

As used herein, the phrase "pharmaceutically acceptable" refers to molecular entities, compositions, materials, excipients, carriers, and the like that do not produce an allergic or similar untoward reaction when administered to humans in general.

As used herein, "poorly soluble" refers to drug having a solubility such that the dose to be administered can be dissolved in 250 ml of aqueous media ranging in pH from 1 to 7.5, a drug with a slow dissolution rate, and a drug with a low equilibrium solubility, for example resulting in decreased bioavailability of the pharmacological effect of the therapeutic drug being delivered.

As used herein, "derivative" refers to chemically modified inhibitors or stimulators that still retain the desired effect or property of the original drug. Such derivatives may be derived by the addition, removal, or substitution of one or more chemical moieties on the parent molecule. Such moieties may include, but are not limited to, an element such as a hydrogen or a halide, or a molecular group such as a methyl group. Such a derivative may be prepared by any method known to those of skill in the art. The properties of such derivatives may be assayed for their desired properties by any means known to those of skill in the art. As used herein, "analogs" include structural equivalents or mimetics.

The solution agent used in the solution can be aqueous such as water, one or more organic solvents, or a combination thereof. When used, the organic solvents can be water miscible or non-water miscible. Suitable organic solvents include but are not limited to ethanol, methanol, tetrahydrofuran, acetonitrile, acetone, tert-butyl alcohol, dimethyl sulfoxide, N,N-dimethyl formamide, diethyl ether, methylene chloride, ethyl acetate, isopropyl acetate, butyl acetate, propyl acetate, toluene, hexanes, heptane, pentane, and combinations thereof.

By "immediate release" is meant a release of an API to an environment over a period of seconds to no more than about 30 minutes once release has begun and release begins within no more than about 2 minutes after administration. An immediate release does not exhibit a significant delay in the release of drug.

By "rapid release" is meant a release of an API to an environment over a period of 1-59 minutes or 0.1 minute to three hours once release has begun and release can begin within a few minutes after administration or after expiration of a delay period (lag time) after administration.

As used herein, the term "extended release" profile assumes the definition as widely recognized in the art of pharmaceutical sciences. An extended release dosage form will release an API at a substantially constant rate over an extended period of time or a substantially constant amount of API will be released incrementally over an extended period of time. An extended release tablet generally effects at least a two-fold reduction in dosing frequency as compared to the API presented in a conventional dosage form (*e.g.*, a solution or rapid releasing conventional solid dosage forms).

By "controlled release" is meant a release of an API to an environment over a period of about eight hours up to about 12 hours, 16 hours, 18 hours, 20 hours, a day, or more than a day. By "sustained release" is meant an extended release of an active agent to maintain a constant drug level in the blood or target tissue of a subject to which the device is administered.

The term "controlled release", as regards to drug release, includes the terms "extended release," "prolonged release," "sustained release," or "slow release," as these terms are used in the pharmaceutical sciences. A controlled release can begin within a few minutes after administration or after expiration of a delay period (lag time) after administration.

A "slow release dosage form" is one that provides a slow rate of release of API so that API is released slowly and approximately continuously over a period of 3 hours, 6 hours, 12 hours, 18 hours, a day, 2 or more days, a week, or 2 or more weeks, for example.

The term "mixed release" as used herein refers to a pharmaceutical agent that includes two or more release profiles for one or more active pharmaceutical ingredients. For example, the mixed release may include an immediate release and an extended release portion, each of which may be the same API or each may be a different API.

A "timed release dosage form" is one that begins to release an API after a predetermined period of time as measured from the moment of initial exposure to the environment of use.

A "targeted release dosage form" generally refers to an oral dosage form that is designed to deliver an API to a particular portion of the gastrointestinal tract of a subject. An exemplary targeted dosage form is an enteric dosage form that delivers a drug into the middle to lower intestinal tract but not into the stomach or mouth of the subject. Other targeted dosage forms can deliver to other sections of the gastrointestinal tract such as the stomach, jejunum, ileum, duodenum, cecum, large intestine, small intestine, colon, or rectum.

By "delayed release" is meant that initial release of an API occurs after expiration of an approximate delay (or lag) period. For example, if release of an API from an extended release composition is delayed two hours, then release of the API begins at about two hours after administration of the composition, or dosage form, to a subject. In general, a delayed release is opposite of an immediate release, wherein release of an API begins after no more than a few minutes after administration. Accordingly, the API release profile from a particular composition can be a delayed-extended release or a delayed-rapid release. A "delayed-extended" release profile is one wherein extended release of an API begins after expiration of an initial delay period. A "delayed-rapid" release profile is one wherein rapid release of an API begins after expiration of an initial delay period.

A "pulsatile release dosage form" is one that provides pulses of high API concentration, interspersed with low concentration troughs. A pulsatile profile containing two peaks may be described as "bimodal." A pulsatile profile of more than two peaks may be described as multimodal.

A "pseudo-first order release profile" is one that approximates a first order release profile. A first order release profile characterizes the release profile of a dosage form that releases a constant percentage of an initial API charge per unit time.

A "pseudo-zero order release profile" is one that approximates a zero-order release profile. A zero-order release profile characterizes the release profile of a dosage form that releases a constant amount of API per unit time.

### II. Processing Methodologies

### A. Thermokinetic Compounding

In certain embodiments, the pharmaceutical formulations of the present disclosure are processed in a thermokinetic chamber as disclosed in U.S. Patent No. 8,486,423, which is incorporated herein by reference. This disclosure is directed to a method of blending certain heat sensitive or thermolabile components in a thermokinetic mixer by using multiple speeds during a single, rotationally continuous operation on a batch containing thermolabile components in order to minimize any substantial thermal degradation, so that the resulting pharmaceutical compositions have increased bioavailability and stability.

In a TKC chamber the average temperature inside the chamber is ramped up to a pre-defined final temperature over the duration of processing to achieve thermokinetic compounding of an API and the one or more pharmaceutically acceptable excipients, adjuvants, additional APIs, or combinations thereof, into a composite. The length of processing and exposure to elevated temperatures during thermokinetic compounding will generally be below the thermal sensitivity threshold of the API, the excipients, the adjuvants, the additional APIs, or all of these. Multiple speeds may be used during a single, rotationally continuous TKC operation to achieve optimal thermokinetic mixing of the API and the one or more pharmaceutically acceptable excipients, adjuvants and additional APIs, or combinations thereof, into a composite with minimal thermal degradation. The pre-defined final temperature and speed(s) are selected to reduce the possibility that the API, excipients, adjuvants, additional APIs and/or processing agents are degraded or their functionality is impaired during processing. Generally, the pre-defined final temperature, pressure, time of processing and other environmental conditions (*e*.*g*., pH, moisture, buffers, ionic strength, O₂) will be selected to substantially eliminate API, excipient, adjuvant, additional APIs and/or processing agent degradation.

One embodiment is a method for continuous blending and melting of an autoheated mixture in the mixing chamber of a high speed mixer, where a first speed is changed mid-processing to a second speed upon achieving a first desired process parameter. Another embodiment is the use of variations in the shape, width and angle of the facial portions of the shaft extensions or projections that intrude into the main processing volume to control translation of rotational shaft energy delivered to the extensions or projections into heating energy within particles impacting the portions of the extensions or projections. Other embodiments include:
producing solid dispersions of the active pharmaceutical ingredient, with or without additional APIs, by processing at low temperatures for very brief durations;
producing solid dispersions of the active pharmaceutical ingredient, with or without additional APIs, in thermolabile polymers and excipients by processing at low temperatures for very brief durations;
rendering the active pharmaceutical ingredient, with or without additional APIs, amorphous while dispersing in a polymeric, non-polymeric, or combination excipient carrier system;
rendering the active pharmaceutical ingredient, with or without additional APIs, amorphous while dispersing in a polymeric, non-polymeric, or combination excipient carrier system and adjuvants;
dry milling of crystalline the active pharmaceutical ingredient to reduce the particle size of the bulk material;
wet milling of crystalline the active pharmaceutical ingredient with a pharmaceutically acceptable solvent to reduce the particle size of the bulk material;
melt milling of crystalline the active pharmaceutical ingredient with one or more molten pharmaceutical excipients having limited miscibility with the crystalline the active pharmaceutical ingredient to reduce the particle size of the bulk material;
milling crystalline the active pharmaceutical ingredient in the presence of polymeric or non-polymeric excipient to create ordered mixtures where fine the active pharmaceutical ingredient particles adhere to the surface of excipient particles and/or excipient particles adhere to the surface of fine the active pharmaceutical ingredient particles;
producing single phase, miscible composites of the active pharmaceutical ingredient and one or more other pharmaceutical materials previously considered to be immiscible for utilization in a secondary processing step, *e.g.* melt extrusion, film coating, tableting and granulation;
pre-plasticizing polymeric materials for subsequent use in film coating or melt extrusion operations;
rendering a crystalline or semi-crystalline pharmaceutical polymer amorphous, which can be used as a carrier for the active pharmaceutical ingredient, in which the amorphous character improves the dissolution rate of the active pharmaceutical ingredient-polymer composite, the stability of the active pharmaceutical ingredient-polymer composite, and/or the miscibility of the active pharmaceutical ingredient and the polymer;
deaggregating and dispersing engineered particles in a polymeric carrier without altering the properties of the engineered particles;
simple blending of the active pharmaceutical ingredient, with or without additional APIs, in powder form with one or more pharmaceutical excipients;
producing composites comprising the active pharmaceutical ingredient, with or without additional APIs, and one or more thermolabile polymers without the use of processing agents; and
homogenously dispersing the active pharmaceutical ingredient, with or without additional APIs, with a coloring agent or opacifying agent within a polymer carrier or excipient blend.

### B. Other Processes

Additionally, compositions of the present disclosure may be processed using any technique known to one skilled in the art to produce a solid formulation, including fusion or solvent based techniques. Specific examples of these techniques include extrusion, melt extrusion, hot-melt extrusion, spray congealing, spray drying, hot-spin mixing, ultrasonic compaction, and electrostatic spinning.

### III. Drug Formulations

### A. Active Pharmaceutical Ingredients

The presently disclosed methods may be applied to any of a wide variety of active pharmaceutical ingredients. However, certain methods are particularly envisioned to employ APIs that are poorly soluble.

### B. Delivery

A variety of administration routes are available for delivering the active pharmaceutical ingredient to a patient in need. The particular route selected will depend upon the particular drug selected, the weight and age of the patient, and the dosage required for therapeutic effect. The pharmaceutical compositions may conveniently be presented in unit dosage form. The active pharmaceutical ingredient suitable for use in accordance with the present disclosure, and its pharmaceutically acceptable salts, derivatives, analogs, prodrugs, and solvates thereof, can be administered alone, but will generally be administered in admixture with a suitable pharmaceutical excipient, adjuvant, diluent, or carrier selected with regard to the intended route of administration and standard pharmaceutical practice, and can in certain instances be administered with one or more additional API(s), preferably in the same unit dosage form.

The active pharmaceutical ingredient may be used in a variety of application modalities, including oral delivery as tablets, capsules or suspensions; pulmonary and nasal delivery; topical delivery as emulsions, ointments or creams; transdermal delivery; and parenteral delivery as suspensions, microemulsions or depot. As used herein, the term "parenteral" includes subcutaneous, intravenous, intramuscular, or infusion routes of administration.

### C. Lubricants

The lubricants as envisioned for applications within the scope of this disclosure are crystalline, poorly water-soluble to insoluble, and non-polymeric. Though starting in a crystalline form, the lubricants are made amorphous during thermokinetic processing. The resulting amorphous lubricant is more water soluble and able to interact with drug in-solution and providing a solubility/bioavailability benefit.

Regarding lubrication agents, although magnesium stearate and stearic acid are the most frequently used lubricants in the pharmaceutical industry, there are other lubricants in use as well. For example, fatty acids, fatty acid esters, metallic salts of fatty acids, as well as inorganic materials and polymers can fill this role.

Lubricants are often classified by their water solubility, *i.e.*, water soluble or insoluble. Selection of the type of lubricant will depend on the type of administration, tablet architecture, desired dissolution and pharmacodynanic properties, and cost. Some water insoluble lubricants include the stearates (magnesium stearate, calcium stearate, sodium stearate), talc, sterotex, waxes, stearowet, glyceryl behapate (Compritol^{®}888) and liquid paraffin. Some water soluble lubricants include boric acid, sodium benzoate, sodium oleate, sodium acetate, and magnesium lauryl sulfate.

Anti-adherents are a subclass of lubricants that counter the strong adhesive properties of some drugs towards metals used in tablet formation, and can prevent sticking. Such agents include talc, cornstarch, colloidal silica, DL-leucine, sodium lauryl sulfate and the stearate molecules mentioned above. Glidants, another subcategory of agents that includes lubricants mentioned above, are used to improve flow properties of materials, include talc, starches, and colloidal silicas (e.g., syloid, pyrogenic silica, hydrated sodium silioaluminate).

### D. Other Excipients

The excipients and adjuvants that may be used in the presently disclosed compositions and composites, while potentially having some activity in their own right, for example, antioxidants, are generally defined for this application as compounds that enhance the efficiency and/or efficacy of the active pharmaceutical ingredient. It is also possible to have more than one API in a given solution, so that the particles formed contain more than one API.

Any pharmaceutically acceptable excipient known to those of skill in the art may be used to produce the composites and compositions disclosed herein. Examples of excipients for use with the present disclosure include, but are not limited to, *e.g.*, a pharmaceutically acceptable polymer, a thermolabile polymeric excipient, or a non-polymeric exicipient. Other non-limiting examples of excipients include, lactose, glucose, starch, calcium carbonate, kaoline, crystalline cellulose, silicic acid, water, simple syrup, glucose solution, starch solution, gelatin solution, carboxymethyl cellulose, shellac, methyl cellulose, polyvinyl pyrrolidone, dried starch, sodium alginate, powdered agar, calcium carmelose, a mixture of starch and lactose, sucrose, butter, hydrogenated oil, a mixture of a quaternary ammonium base and sodium lauryl sulfate, glycerine and starch, lactose, bentonite, colloidal silicic acid, talc, stearates, and polyethylene glycol, sorbitan esters, polyoxyethylene sorbitan fatty acid esters, polyoxyethylene alkyl ethers, poloxamers (polyethylene-polypropylene glycol block copolymers), sucrose esters, sodium lauryl sulfate, oleic acid, lauric acid, vitamin E TPGS, polyoxyethylated glycolysed glycerides, dipalmitoyl phosphadityl choline, glycolic acid and salts, deoxycholic acid and salts, sodium fusidate, cyclodextrins, polyethylene glycols, polyglycolyzed glycerides, polyvinyl alcohols, polyacrylates, polymethacrylates, polyvinylpyrrolidones, phosphatidyl choline derivatives, cellulose derivatives, biocompatible polymers selected from poly(lactides), poly(glycolides), poly(lactide-co-glycolides), poly(lactic acid)s, poly(glycolic acid)s, poly(lactic acid-co-glycolic acid)s and blends, combinations, and copolymers thereof.

As stated, excipients and adjuvants may be used to enhance the efficacy and efficiency of the API. Additional non-limiting examples of compounds that can be included are binders, carriers, cryoprotectants, lyoprotectants, surfactants, fillers, stabilizers, polymers, protease inhibitors, antioxidants, bioavailability enhancers and absorption enhancers. The excipients may be chosen to modify the intended function of the active ingredient by improving flow, or bio-availability, or to control or delay the release of the API. Specific nonlimiting examples include: sucrose, trehaolose, Span 80, Span 20, Tween 80, Brij 35, Brij 98, Pluronic, sucroester 7, sucroester 11, sucroester 15, sodium lauryl sulfate (SLS, sodium dodecyl sulfate. SDS), dioctyl sodium sulphosuccinate (DSS, DOSS, dioctyl docusate sodium), oleic acid, laureth-9, laureth-8, lauric acid, vitamin E TPGS, Cremophor^{®} EL, Cremophor^{®} RH, Gelucire^{®} 50/13, Gelucire^{®} 53/10, Gelucire^{®} 44/14, Labrafil^{®}, Solutol^{®} HS, dipalmitoyl phosphadityl choline, glycolic acid and salts, deoxycholic acid and salts, sodium fusidate, cyclodextrins, polyethylene glycols, Labrasol^{®}, polyvinyl alcohols, polyvinyl pyrrolidones and tyloxapol. Using the process of the present disclosure, the morphology of the active ingredients can be modified, resulting in highly porous microparticles and nanoparticles.

Exemplary polymer carriers or thermal binders that may be used in the presently disclosed compositions and composites include but are not limited to polyethylene oxide; polypropylene oxide; polyvinylpyrrolidone; polyvinylpyrrolidone-co-vinylacetate; acrylate and methacrylate copolymers; polyethylene; polycaprolactone; polyethylene-co-polypropylene; alkylcelluloses such as methylcellulose; hydroxyalkylcelluloses such as hydroxymethylcellulose, hydroxyethylcellulose, hydroxypropylcellulose, and hydroxybutylcellulose; hydroxyalkyl alkylcelluloses such as hydroxyethyl methylcellulose and hydroxypropyl methylcellulose; starches, pectins; polysaccharides such as tragacanth, gum arabic, guar gum, and xanthan gum. One embodiment of the binder is poly(ethylene oxide) (PEO), which can be purchased commercially from companies such as the Dow Chemical Company, which markets PEO under the POLY OX^{®} exemplary grades of which can include WSR N80 having an average molecular weight of about 200,000; 1,000,000; and 2,000,000.

Suitable polymer carriers or thermal binders that may or may not require a plasticizer include, for example, Eudragit^{®} RS PO, Eudragit^{®} S100, Kollidon^{®} SR (poly(vinyl acetate)-co-poly(vinylpyrrolidone) copolymer), Ethocel^{®} (ethylcellulose), HPC (hydroxypropylcellulose), cellulose acetate butyrate, poly(vinylpyrrolidone) (PVP), poly(ethylene glycol) (PEG), poly(ethylene oxide) (PEO), poly(vinyl alcohol) (PVA), hydroxypropyl methylcellulose (HPMC), ethylcellulose (EC), hydroxyethylcellulose (HEC), sodium carboxymethyl-cellulose (CMC), dimethylaminoethyl methacrylate-methacrylic acid ester copolymer, ethylacrylate-methylmethacrylate copolymer (GA-MMA), C-5 or 60 SH-50 (Shin-Etsu Chemical Corp.), cellulose acetate phthalate (CAP), cellulose acetate trimelletate (CAT), poly(vinyl acetate) phthalate (PVAP), hydroxypropylmethylcellulose phthalate (HPMCP), poly(methacrylate ethylacrylate) (1:1) copolymer (MA-EA), poly(methacrylate methylmethacrylate) (1:1) copolymer (MA-MMA), poly(methacrylate methylmethacrylate) (1:2) copolymer, Eudragit^{®} L-30-D (MA-EA, 1:1), Eudragit^{®} L100-55 (MA-EA, 1:1), Eudragit^{®} EPO (poly(butyl methacylate-co-(2-dimethylaminoethyl) methacrylate-co-methyl methacrylate) 1:2:1), hydroxypropylmethylcellulose acetate succinate (HPMCAS), Coateric^{®} (PVAP), Aquateric^{®} (CAP), and AQUACOAT^{®} (HPMCAS), Soluplus^{®} (polyvinyl caprolactam-polyvinyl acetate-polyethylene glycol graft copolymer, BASF), Luvitec^{®} K 30 (polyvinylpyrrolidone, PVP), Kollidon^{®} (polyvinylpyrrolidone, PVP), polycaprolactone, starches, pectins; polysaccharides such as tragacanth, gum arabic, guar gum, and xanthan gum.

The stabilizing and non-solubilizing carrier may also contain various functional excipients, such as: hydrophilic polymer, antioxidant, super-disintegrant, surfactant including amphiphilic molecules, wetting agent, stabilizing agent, retardant, similar functional excipient, or combination thereof, and plasticizers including citrate esters, polyethylene glycols, PG, triacetin, diethylphthalate, castor oil, and others known to those or ordinary skill in the art. Extruded material may also include an acidifying agent, adsorbent, alkalizing agent, buffering agent, colorant, flavorant, sweetening agent, diluent, opaquant, complexing agent, fragrance, preservative or a combination thereof.

Exemplary hydrophilic polymers which can be a primary or secondary polymeric carrier that can be included in the composites or composition disclosed herein include poly(vinyl alcohol) (PVA), polyethylene-polypropylene glycol (*e*.*g*., POLOXAMER^{®}), carbomer, polycarbophil, or chitosan. Hydrophilic polymers for use with the present disclosure may also include one or more of hydroxypropyl methylcellulose, carboxymethylcellulose, hydroxypropyl cellulose, hydroxyethyl cellulose, methylcellulose, natural gums such as gum guar, gum acacia, gum tragacanth, or gum xanthan, and povidone. Hydrophilic polymers also include polyethylene oxide, sodium carboxymethycellulose, hydroxyethyl methyl cellulose, hydroxymethyl cellulose, carboxypolymethylene, polyethylene glycol, alginic acid, gelatin, polyvinyl alcohol, polyvinylpyrrolidones, polyacrylamides, polymethacrylamides, polyphosphazines, polyoxazolidines, poly(hydroxyalkylcarboxylic acids), carrageenate alginates, carbomer, ammonium alginate, sodium alginate, or mixtures thereof.

Compositions with enhanced solubility may comprise a mixture of the active pharmaceutical ingredient and an additive that enhances the solubility of the active pharmaceutical ingredient. Examples of such additives include but are not limited to surfactants, polymer carriers, pharmaceutical carriers, thermal binders or other excipients. A particular example may be a mixture of the active pharmaceutical ingredient with a surfactant or surfactants, the active pharmaceutical ingredient with a polymer or polymers, or the active pharmaceutical ingredient with a combination of a surfactant and polymer carrier or surfactants and polymer carriers. A further example is a composition where the active pharmaceutical ingredient is a derivative or analog thereof.

Surfactants that can be used in the disclosed compositions to enhance solubility have been previously presented. Particular examples of such surfactants include but are not limited to sodium dodecyl sulfate, dioctyl docusate sodium, Tween 80, Span 20, Cremophor^{®} EL or Vitamin E TPGS. Polymer carriers that can be used in the disclosed composition to enhance solubility have been previously presented. Particular examples of such polymer carriers include but are not limited to Soluplus^{®}, Eudragit^{®} L100-55, Eudragit^{®} EPO, Kollidon^{®} VA 64, Luvitec^{®}. K 30, Kollidon^{®}, AQOAT^{®}-HF, and AQOAT^{®}-LF. The composition of the present disclosure can thus be any combination of one or more of the APIs, zero, one or more of surfactants or zero, one or more of polymers presented herein.

Solubility can be indicated by peak solubility, which is the highest concentration reached of a species of interest over time during a solubility experiment conducted in a specified medium. The enhanced solubility can be represented as the ratio of peak solubility of the agent in a pharmaceutical composition of the present disclosure compared to peak solubility of the reference standard agent under the same conditions. Preferable, an aqueous buffer with a pH in the range of from about pH 4 to pH 8, about pH 5 to pH 8, about pH 6 to pH 7, about pH 6 to pH 8, or about pH 7 to pH 8, such as, for example, pH 4.0, 4.5, 5.0, 5.5, 6.0, 6.2, 6.4, 6.6, 6.7, 6.8, 6.9, 7.0, 7.1, 7.2, 7.4, 7.6, 7.8, or 8.0, may be used for determining peak solubility. This peak solubility ratio can be about 2:1, 3:1, 4:1, 5:1, 6:1, 7:1, 8:1, 9:1, 10:1, 12:1, 15:1, 20:1, 25:1, 30:1, 35:1, 40:1, 45:1, 50:1, 55:1 or higher.

Compositions of the active pharmaceutical ingredient that enhance bioavailability may comprise a mixture of the active pharmaceutical ingredient and one or more pharmaceutically acceptable adjuvants that enhance the bioavailability of the active pharmaceutical ingredient. Examples of such adjuvants include but are not limited to enzymes inhibitors. Particular examples are such enzyme inhibitors include but are not limited to inhibitors that inhibit cytochrome P-450 enzyme and inhibitors that inhibit monoamine oxidase enzyme. Bioavailability can be indicated by the Cₘₐₓ of the active pharmaceutical ingredient as determined during *in vivo* testing, where Cₘₐₓ is the highest reached blood level concentration of the active pharmaceutical ingredient over time of monitoring. Enhanced bioavailability can be represented as the ratio of Cₘₐₓ of the active pharmaceutical ingredient in a pharmaceutical composition of the present disclosure compared to Cₘₐₓ of the reference standard the active pharmaceutical ingredient under the same conditions. This Cₘₐₓ ratio reflecting enhanced bioavailability can be about 5:1, 6:1, 7:1, 8:1, 9:1, 10:1, 12:1, 15:1, 20:1, 25:1, 30:1, 35:1, 40:1, 45:1, 50:1, 55:1, 60:1, 65:1, 70:1, 75:1, 80:1, 85:1, 90:1, 95:1, 98:1, 99:1, 100:1 or higher.

### IV. EXAMPLES

It will be understood that particular embodiments described herein are shown by way of illustration and not as limitations of the disclosure. The principal features of this disclosure can be employed in various embodiments without departing from the scope of the disclosure. All of the compositions and/or methods disclosed and claimed herein can be made and executed without undue experimentation in light of the present disclosure. While the compositions and methods of this disclosure have been described in terms of preferred embodiments, it will be apparent to those of skill in the art that variations may be applied to the compositions and/or methods and in the steps or in the sequence of steps of the method described herein without departing from the concept, spirit and scope of the disclosure. All such similar substitutes and modifications apparent to those skilled in the art are deemed to be within the spirit, scope and concept of the disclosure as defined by the appended claims.

### Example 1

Lubricants as defined above may be added to the processing methods as a processing aid to improve yield when processing by thermo-kinetic mixing. For example, amorphous compositions were prepared containing vemurafenib (active pharmaceutical ingredient), pharmaceutical polymer (hypromellose acetate succinate), and with/without 0.5% lubricant (sodium stearyl fumarate). In an *in vitro* dissolution test, solubility performance was improved significantly for the composition containing sodium stearyl fumarate.

In another example, amorphous compositions were prepared containing deferasirox (active pharmaceutical ingredient), pharmaceutical polymers (methacrylic acid and vinylpyrrolidone-vinyl acetate copolymers), and with/without 0.4% lubricant (magnesium stearate). These amorphous compositions were formulated into final tablets (see Table 1) and comparatively evaluated for pharmacokinetic performance in an *in vivo* dog model. From this study it was determined that bioavailability was improved significantly for the compositions containing magnesium stearate inside the amorphous dispersion relative to the same compositions not containing magnesium stearate in the amorphous dispersion (see Table 2 and FIG. 1).

**TABLE 1 - Formulation Information***

| **Phase** | **Component** | **Lot 25** | **Lot 52** | **Lot 28** | **Lot 53** |
|---|---|---|---|---|---|
| Amorphous Intermediate | Deferasirox | 40% | 40% | 40% | 40% |
| | Copovidone | 20% | 19.8% | 40% | 39.6% |
| | Methacrylic Acid and Ethyl Acrylate Copolymer | 20% | 19.8% | 0% | 0% |
| | Magnesium Stearate | 0% | 0.4% | 0% | 0.4% |
| External /tableting | Microcrystalline Cellulose | 13% | 13% | 13% | 13% |
| | Croscarmellose Sodium | 6% | 6% | 6% | 6% |
| | Colloidal Silicon Dioxide | 0.5% | 0.5% | 0.5% | 0.5% |
| | Magnesium Stearate | 0.5% | 0.5% | 0.5% | 0.5% |

| | | | | | |
|---|---|---|---|---|---|
| * - All tablets prepared 900 mg total weight with 360 mg of deferasirox (active pharmaceutical ingredient); amorphous intermediate prepared by thermo-kinetic mixing | | | | | |

**TABLE 2- PK Data from Dog Study**

| **PK Parameter** | **Lot 25** | **Lot 52** | **Lot 28** | **Lot 53** |
|---|---|---|---|---|
| AUC (ng*hr/ml) | 262,333±61,028 | 394,815±101,967 | 283,375±39,668 | 409,369±133,071 |
| CMax (ng/ml) | 48,550±17,337 | 75,750±25,364 | 59,775±5,480 | 88,300±32,129 |
| TMax (hr) | 1.75±0.29 | 2.13±0.63 | 2.00±0.82 | 1.50±0.41 |

### Example 2

In another example, thermokinetic compounding was performed on compositions of itraconazole (active pharmaceutical ingredient), various grades of hypromellose (pharmaceutical polymer), and magnesium stearate (lubricant). These compositions are summarized in Table 3. Batch 17-1 utilizes hypromellose 2910, 5 cps as the polymer carrier. Batch 17-2 utilizes hypromellose 2910 E5 as the polymer carrier and contains the addition of 2% magnesium stearate (MgSt) as a lubricant. Batch 17-3 utilizes hypromellose 2910 E15 (HPMC E15) as the polymer carrier. Batch 17-4 utilizes hypromellose 2910 E15 as the polymer carrier and contains the addition of 2% magnesium stearate (MgSt) as a lubricant. Batch 17-5 utilizes hypromellose 2910 E50 (HPMC E50) as the polymer carrier. Batch 17-6 utilizes hypromellose 2910 E50 as the polymer carrier and contains the addition of 2% magnesium stearate (MgSt) as a lubricant.

The processing parameters and temperature versus time profiles for thermokinetic compounding of batches 17-1 through 17-6 are provided in FIG. 2. This figure signifies that the target amorphous dispersions were achieved by thermokinetic compounding at a peak temperature below the melting point of itraconazole and with a time at elevated temperature of less than 20 seconds. Both the low temperature and brief processing duration are critical to producing the amorphous dispersion without degradation to the drug and/or polymer.

Batches 17-1 through 17-6 were analyzed for crystalline content by x-ray powder diffraction (XRPD). The results of the analysis are provided in FIG. 3. These results demonstrate that these compositions of active pharmaceutical ingredient, pharmaceutical polymer, and lubricant were rendered amorphous by the process across a range of pharmaceutical polymer grades.

**TABLE 3 - Formulation Table for Variable Hypromellose Compositions***

| **Batch number** | **Itraconazole** | **HPMC E5** | **HPMC E15** | **HPMC E50** | **MgSt** |
|---|---|---|---|---|---|
| ITZ.20170417-1 | 33.3% | 66.7% | | - | |
| ITZ.20140417-2 | 33.3% | 64.7% | | | 2.0% |
| ITZ.20140417-3 | 33.3% | | 66.7% | | |
| ITZ.20140417-4 | 33.3% | | 64.7% | | 2.0% |
| ITZ.20140417-5 | 33.3% | | | 66.7% | |
| ITZ.20140417-6 | 33.3% | | | 64.7% | 2.0% |

| | | | | | |
|---|---|---|---|---|---|
| * - All batches contained 33.3% itraconazole as the active pharmaceutical ingredient. Batches 1 and 2 utilized hypromellose 2910 E5 as the polymer carrier. Batches 3 and 4 utilized hypromellose 2910 E15 as the polymer carrier. Batches 5 and 6 utilized hypromellose 2910 E50 as the polymer carrier. Batches 2, 4, and 6 contained 2% magnesium stearate as a lubricant. | | | | | |

### Example 3

In another example, thermokinetic compounding was performed on compositions of itraconazole (active pharmaceutical ingredient), hypromellose 2910 E15 (pharmaceutical polymer), and various lubricants. These compositions are summarized in Table 4. Batch 28-1 contains the addition of 2% sodium stearyl fumarate (SSF) as a lubricant. Batch 28-2 contains the addition of 2% glyceryl monostearate (GMS) as a lubricant. Batch 28-3 contains the addition of 2% stearic acid (SA) as a lubricant. Batch 28-4 contains the addition of 2% myristic acid (MA) as a lubricant.

The processing parameters and temperature versus time profiles for thermokinetic compounding of batches 28-1 through 28-4 are provided in FIG. 4. This figure signifies that the target amorphous dispersions were achieved by thermokinetic compounding at a peak temperature below the melting point of itraconazole and with a time at elevated temperature of less than 10 seconds. Both the low temperature and brief processing duration are critical to producing the amorphous dispersion without degradation to the drug and/or polymer.

Batches 28-1 through 28-4 were analyzed for crystalline content by x-ray powder diffraction (XRPD). The results of the analysis are provided in FIG. 5. These results demonstrate that these compositions of active pharmaceutical ingredient, pharmaceutical polymer, and lubricant were rendered amorphous by the process across of a range of lubricants selected.

The *in vitro* dissolution results generated for the compositions described in this example, and the preceding one, did not show performance enhancement with the inclusion of the investigated lubricants. This signifies the importance of broadly screening a variety of lubricant excipients to identify those that are able interact with the drug of interest to increase aqueous solution concentrations. Considering the complexity of the system with respect to intermolecular interactions between each of the components of the composition (drug, polymer, lubricant), and the complexity of each of these with the molecules comprising the aqueous dissolution medium, the properties of a lubricant excipient that lead to solubility/dissolution enhancement of the drug cannot be determined *a priori*, and thus must be determined via empirical observation. Hence, in the specific cases of Examples 2 and 3 herein, additional testing is required to identify a lubricant that yields a positive interaction with itraconazole to elevate its aqueous solution concentrations.

**TABLE 4 - Formulation Table for Variable Lubricant Compositions***

| **Batch number** | **Itraconazole** | **HPMC E15** | **SSF** | **GMS** | **SA** | **MA** |
|---|---|---|---|---|---|---|
| ITZ.20170428-1 | 33.3% | 64.7% | 2.0% | | | |
| ITZ.20140428-2 | 33.3% | 64.7% | | 2.0% | | |
| ITZ.20140428-3 | 33.3% | 64.7% | | | 2.0% | |
| ITZ.20140428-4 | 33.3% | 64.7% | | | | 2.0% |

| | | | | | | |
|---|---|---|---|---|---|---|
| * - All batches contained 33.3% itraconazole as the active pharmaceutical ingredient and 64.7% hypromellose 2910 E15 as the pharmaceutical polymer. Batch 1 contained 2% sodium stearyl fumarate as a lubricant. Batch 2 contained 2% glyceryl monostearate as a lubricant. Batch 3 contained 2% stearic acid as a lubricant. Batch 4 contained 2% myristic acid as a lubricant. | | | | | | |

All of the compositions and methods disclosed and claimed herein can be made and executed without undue experimentation in light of the present disclosure. While the compositions and methods of this disclosure have been described in terms of preferred embodiments, it will be apparent to those of skill in the art that variations may be applied to the compositions and methods, and in the steps or in the sequence of steps of the methods described herein without departing from the concept, spirit and scope of the disclosure. More specifically, it will be apparent that certain agents which are both chemically and physiologically related may be substituted for the agents described herein while the same or similar results would be achieved. All such similar substitutes and modifications apparent to those skilled in the art are deemed to be within the spirit, scope and concept of the disclosure as defined by the appended claims.
Particularly preferred embodiments of the invention are set forth in items 1 to 53 below:
1. A method of making a pharmaceutical composition comprising:
   (a) providing the active pharmaceutical ingredient and one or more pharmaceutically acceptable excipients including a non-polymeric lubricant;
   (b) processing the materials of step (a) using thermal processing or solvent evaporation,
   wherein the processing of the active pharmaceutical ingredient and the one or more pharmaceutically acceptable excipients including a non-polymeric lubricant forms an amorphous pharmaceutical composition.
2. The method of item 1, wherein said pharmaceutical comprises more than one active pharmaceutical ingredient.
3. The method of item 1, wherein the more than one pharmaceutically acceptable excipient comprises a surfactant.
4. The method of item 1, wherein the more than one pharmaceutically acceptable excipient comprises a pharmaceutical polymer.
5. The method of item 1, wherein the more than one pharmaceutically acceptable excipient comprises one or more surfactants and one or more polymer carriers.
6. The method of item 1, wherein the more than one pharmaceutically acceptable excipient comprises an agent selected from the group consisting of poly(vinyl acetate)-co-poly(vinylpyrrolidone) copolymer, ethylcellulose, hydroxypropylcellulose, cellulose acetate butyrate, poly(vinylpyrrolidone), poly(ethylene glycol), poly(ethylene oxide), poly(vinyl alcohol), hydroxypropyl methylcellulose, ethylcellulose, hydroxyethylcellulose, sodium carboxymethyl-cellulose, dimethylaminoethyl methacrylate-methacrylic acid ester copolymer, ethylacrylate-methylmethacrylate copolymer, cellulose acetate phthalate, cellulose acetate trimelletate, poly(vinyl acetate) phthalate, hydroxypropylmethylcellulose phthalate, poly(methacrylate ethylacrylate) (1:1) copolymer, poly(methacrylate methylmethacrylate) (1:1) copolymer, poly(methacrylate methylmethacrylate) (1:2) copolymer, hydroxypropylmethylcellulose acetate succinate and polyvinyl caprolactam-polyvinyl acetate-polyethylene glycol graft copolymer, carbomer, crospovidone, croscarmellose sodium, sodium dodecyl sulfate, dioctyl sodium sulphosuccinate, polyoxyethylene (20) sorbitan monooleate, glycerol polyethylene glycol oxystearate-fatty acid glycerol polyglycol esters-polyethylene glycols-glycerol ethoxylate, glycerol-polyethylene glycol ricinoleate-fatty acid esters of polyethyleneglycol-polyethylene glycols-ethoxylated glycerol, vitamin E TPGS and sorbitan laurate.
7. The method of item 4, wherein the more than one pharmaceutical polymer comprises an agent selected from the group consisting of poly(vinyl acetate)-co-poly(vinylpyrrolidone) copolymer, ethylcellulose, hydroxypropylcellulose, cellulose acetate butyrate, poly(vinylpyrrolidone), poly(ethylene glycol), poly(ethylene oxide), poly(vinyl alcohol), hydroxypropyl methylcellulose, ethylcellulose, hydroxyethylcellulose, sodium carboxymethyl-cellulose, dimethylaminoethyl methacrylate-methacrylic acid ester copolymer, ethylacrylate-methylmethacrylate copolymer, cellulose acetate phthalate, cellulose acetate trimelletate, poly(vinyl acetate) phthalate, hydroxypropylmethylcellulose phthalate, poly(methacrylate ethylacrylate) (1:1) copolymer, poly(methacrylate methylmethacrylate) (1:1) copolymer, poly(methacrylate methylmethacrylate) (1:2) copolymer, hydroxypropylmethylcellulose acetate succinate and polyvinyl caprolactam-polyvinyl acetate-polyethylene glycol graft copolymer, carbomer, crospovidone, or croscarmellose sodium.
8. The method of item 3, wherein the surfactant comprises an agent selected from the group consisting of sodium dodecyl sulfate, dioctyl sodium sulphosuccinate, polyoxyethylene (20) sorbitan monooleate, glycerol polyethylene glycol oxystearate-fatty acid glycerol polyglycol esters-polyethylene glycols-glycerol ethoxylate, glycerol-polyethylene glycol ricinoleate-fatty acid esters of polyethyleneglycol-polyethylene glycols-ethoxylated glycerol, vitamin E TPGS, and sorbitan laurate, and the pharmaceutical polymer comprises an agent selected from a group consisting of poly(vinylpyrrolidone), ethylacrylate-methylmethacrylate copolymer, poly(methacrylate ethylacrylate) (1:1) copolymer, hydroxypropylmethylcellulose acetate succinate, poly(butyl methacylate-co-(2-dimethylaminoethyl) methacrylate-co-methyl methacrylate) 1:2:1 and polyvinyl caprolactam-polyvinyl acetate-polyethylene glycol graft copolymer.
9. The method of item 1, wherein the active pharmaceutical ingredient is not vemurafenib.
10. The method of item 1, wherein the non-polymeric lubricant comprises and agent selected from magnesium stearate, glyceryl behenate, calcium stearate, (sodium) stearyl fumarate, glyceryl monostearate, glyceryl palmitostearate, myristic acid, palmitic acid, stearic acid, or zinc stearate.
11. The method of item 1, wherein the more than one pharmaceutically acceptable excipients comprises a processing agent, such as a plasticizer.
12. The method of item 1, wherein step (b) is performed at a maximum temperature of about 250 °C, about 225 °C, about 200 °C, about 180 °C, about 150 °C or about 150°C to 250 °C.
13. The method of item 1, wherein the more than one pharmaceutically acceptable excipients comprises a pharmaceutical polymer of high melt viscosity.
14. The method of item 1, wherein the more than one pharmaceutically acceptable excipients comprises a thermally labile pharmaceutical polymer.
15. The method of items 1-14, wherein thermal processing comprises hot melt extrusion or thermokinetic processing.
16. The method of item 1, wherein the active pharmaceutical ingredient to pharmaceutical excipient ratio is about 1 to 4.
17. The method of item 1, wherein the active pharmaceutical ingredient to pharmaceutical excipient ratio is about 3 to 7.
18. The method of item 1, wherein the active pharmaceutical ingredient to pharmaceutical excipient ratio is about 2 to 3.
19. The method of item 1, wherein the active pharmaceutical ingredient to pharmaceutical excipient ratio is about 1 to 1.
20. The method of items 1-19, wherein the non-polymeric lubricant is poorly water soluble or water insoluble and/or crystalline prior to compounding with said active pharmaceutical ingredient.
21. A pharmaceutical composition comprising an amorphous dispersion of the active pharmaceutical ingredient, one or more pharmaceutically acceptable excipients, and a non-polymeric lubricant.
22. The pharmaceutical composition of item 21, wherein said pharmaceutical comprises more than one active pharmaceutical ingredient.
23. The pharmaceutical composition of item 21, wherein the one or more pharmaceutically acceptable excipient comprises a surfactant.
24. The pharmaceutical composition of item 21, wherein the one or more pharmaceutically acceptable excipient comprises a pharmaceutical polymer.
25. The pharmaceutical composition of item 21, wherein the one or more pharmaceutically acceptable excipient comprises a plasticizer.
26. The pharmaceutical composition of item 21, wherein the pharmaceutically acceptable excipient comprises an agent selected from the group consisting of poly(vinyl acetate)-co-poly(vinylpyrrolidone) copolymer, ethylcellulose, hydroxypropylcellulose, cellulose acetate butyrate, poly(vinylpyrrolidone), poly(ethylene glycol), poly(ethylene oxide), poly(vinyl alcohol), hydroxypropyl methylcellulose, ethylcellulose, hydroxyethylcellulose, sodium carboxymethyl-cellulose, dimethylaminoethyl methacrylate-methacrylic acid ester copolymer, ethylacrylate-methylmethacrylate copolymer, cellulose acetate phthalate, cellulose acetate trimelletate, poly(vinyl acetate) phthalate, hydroxypropylmethylcellulose phthalate, poly(methacrylate ethylacrylate) (1:1) copolymer, poly(methacrylate methylmethacrylate) (1:1) copolymer, poly(methacrylate methylmethacrylate) (1:2) copolymer, hydroxypropylmethylcellulose acetate succinate, polyvinyl caprolactam-polyvinyl acetate-polyethylene glycol graft copolymer, carbomer, crospovidone, croscarmellose sodium, sodium dodecyl sulfate, dioctyl sodium sulphosuccinate, polyoxyethylene (20) sorbitan monooleate, glycerol polyethylene glycol oxystearate-fatty acid glycerol polyglycol esters-polyethylene glycols-glycerol ethoxylate, glycerol-polyethylene glycol ricinoleate-fatty acid esters of polyethyleneglycol-polyethylene glycols-ethoxylated glycerol, vitamin E TPGS, and sorbitan laurate.
27. The pharmaceutical composition of item 24, wherein the pharmaceutical polymer comprises an agent selected from the group consisting of poly(vinyl acetate)-co-poly(vinylpyrrolidone) copolymer, ethylcellulose, hydroxypropylcellulose, cellulose acetate butyrate, poly(vinylpyrrolidone), poly(ethylene glycol), poly(ethylene oxide), poly(vinyl alcohol), hydroxypropyl methylcellulose, ethylcellulose, hydroxyethylcellulose, sodium carboxymethyl-cellulose, dimethylaminoethyl methacrylate-methacrylic acid ester copolymer, ethylacrylate-methylmethacrylate copolymer, cellulose acetate phthalate, cellulose acetate trimelletate, poly(vinyl acetate) phthalate, hydroxypropylmethylcellulose phthalate, poly(methacrylate ethylacrylate) (1:1) copolymer, poly(methacrylate methylmethacrylate) (1:1) copolymer, poly(methacrylate methylmethacrylate) (1:2) copolymer, hydroxypropylmethylcellulose acetate succinate and polyvinyl caprolactam-polyvinyl acetate-polyethylene glycol graft copolymer, carbomer, crospovidone, or croscarmellose sodium.
28. The pharmaceutical composition of item 23, wherein the surfactant comprises an agent selected from the group consisting of sodium dodecyl sulfate, dioctyl sodium sulphosuccinate, polyoxyethylene (20) sorbitan monooleate, glycerol polyethylene glycol oxystearate-fatty acid glycerol polyglycol esters-polyethylene glycols-glycerol ethoxylate, glycerol-polyethylene glycol ricinoleate-fatty acid esters of polyethyleneglycol-polyethylene glycols-ethoxylated glycerol, vitamin E TPGS, and sorbitan laurate, and the pharmaceutical polymer comprises an agent selected from a group consisting of poly(vinylpyrrolidone), hydroxypropylcellulose, poly(vinyl alcohol), hydroxypropyl methylcellulose, hydroxyethylcellulose, and sodium carboxymethyl-cellulose.and polyvinyl caprolactam-polyvinyl acetate-polyethylene glycol graft copolymer.
29. The pharmaceutical composition of item 21, wherein said pharmaceutical composition does not contain a processing agent.
30. The pharmaceutical composition of item 21, wherein said pharmaceutical composition does not contain a plasticizer.
31. The pharmaceutical composition of item 21, wherein the active pharmaceutical ingredient to pharmaceutical excipient ratio is about 1 to 4.
32. The pharmaceutical composition of item 21, wherein the active pharmaceutical ingredient to pharmaceutical excipient ratio is about 3 to 7.
33. The pharmaceutical composition of item 21, wherein the active pharmaceutical ingredient to pharmaceutical excipient ratio is about 2 to 3.
34. The pharmaceutical composition of item 21, wherein the active pharmaceutical ingredient to pharmaceutical polymer ratio is about 1 to 1.
35. The pharmaceutical composition of item 21, wherein the one or more pharmaceutically acceptable excipients comprises a pharmaceutical polymer of high melt viscosity.
36. The pharmaceutical composition of item 21, wherein the one or more pharmaceutically acceptable excipients comprises a thermally labile pharmaceutical polymer.
37. The pharmaceutical composition of item 21, wherein peak solubility of the active pharmaceutical ingredient and the reference standard the active pharmaceutical ingredient in an aqueous buffer with a pH range of 4 to 8 have a ratio of greater than 3:1.
38. The pharmaceutical composition of item 21, wherein peak solubility of the active pharmaceutical ingredient and the reference standard the active pharmaceutical ingredient in an aqueous buffer with a pH range of 4 to 8 have a ratio of greater than 10:1.
39. The pharmaceutical composition of item 21, wherein peak solubility of the active pharmaceutical ingredient and the reference standard the active pharmaceutical ingredient in an aqueous buffer with a pH range of 4 to 8 have a ratio of greater than 20:1.
40. The pharmaceutical composition of item 21, wherein peak solubility of the active pharmaceutical ingredient and the reference standard the active pharmaceutical ingredient in an aqueous buffer with a pH range of 4 to 8 have a ratio of greater than 30:1.
41. The pharmaceutical composition of item 21, wherein in the Cₘₐₓ of the active pharmaceutical ingredient in the composition and Cₘₐₓ of the reference standard the active pharmaceutical ingredient have a ratio that is greater than 6:1.
42. The pharmaceutical composition of item 21, formulated into an oral dosage form.
43. The pharmaceutical composition of item 42, wherein the oral dosage form is a tablet, a capsule, or a sachet.
44. The pharmaceutical composition of item 21, wherein the active pharmaceutical ingredient is not vemurafenib.
45. The pharmaceutical composition of item 21, wherein the non-polymeric lubricant is an agent selected from magnesium stearate, glyceryl behenate, calcium stearate, (sodium) stearyl fumarate, glyceryl monostearate, glyceryl palmitostearate, myristic acid, palmitic acid, stearic acid, or zinc stearate.
46. The pharmaceutical composition of items 21-45, wherein the non-polymeric lubricant is poorly water soluble or water insoluble and/or crystalline prior to compounding with said active pharmaceutical ingredient.
47. A pharmaceutical composition produced by a process comprising the steps of:
   (a) providing an active pharmaceutical ingredient and one or more pharmaceutically acceptable excipients including a non-polymeric lubricant;
   (b) processing the materials of step (a) using thermal processing or solvent evaporation
   wherein the processing of the active pharmaceutical ingredient and the one or more pharmaceutically acceptable excipients including a non-polymeric lubricant forms an amorphous pharmaceutical composition.
48. The pharmaceutical composition of item 47, wherein said more than one or more pharmaceutically acceptable excipients comprises an agent selected from the group consisting of poly(vinyl acetate)-co-poly(vinylpyrrolidone) copolymer, ethylcellulose, hydroxypropylcellulose, cellulose acetate butyrate, poly(vinylpyrrolidone), poly(ethylene glycol), poly(ethylene oxide), poly(vinyl alcohol), hydroxypropyl methylcellulose, ethylcellulose, hydroxyethylcellulose, sodium carboxymethyl-cellulose, dimethylaminoethyl methacrylate-methacrylic acid ester copolymer, ethylacrylate-methylmethacrylate copolymer, cellulose acetate phthalate, cellulose acetate trimelletate, poly(vinyl acetate) phthalate, hydroxypropylmethylcellulose phthalate, poly(methacrylate ethylacrylate) (1:1) copolymer, poly(methacrylate methylmethacrylate) (1:1) copolymer, poly(methacrylate methylmethacrylate) (1:2) copolymer, hydroxypropylmethylcellulose acetate succinate and polyvinyl caprolactam-polyvinyl acetate-polyethylene glycol graft copolymer sodium dodecyl sulfate, dioctyl sodium sulphosuccinate, polyoxyethylene (20) sorbitan monooleate, glycerol polyethylene glycol oxystearate-fatty acid glycerol polyglycol esters-polyethylene glycols-glycerol ethoxylate, glycerol-polyethylene glycol ricinoleate-fatty acid esters of polyethyleneglycol-polyethylene glycols-ethoxylated glycerol, vitamin E TPGS and sorbitan laurate.
49. The pharmaceutical composition of item 47, wherein said pharmaceutical composition comprises a processing agent, such as a plasticizer.
50. The pharmaceutical formulation of item 47, wherein said active pharmaceutical ingredient is not vemurafenib.
51. The pharmaceutical formulation of item 47, wherein said lubricant is an agent selected from, magnesium stearate, glyceryl behenate, calcium stearate, (sodium) stearyl fumarate, glyceryl monostearate, glyceryl palmitostearate, myristic acid, palmitic acid, stearic acid, or zinc stearate.
52. The pharmaceutical formulation of item 47, wherein thermal processing comprises hot melt extrusion or thermokinetic processing.
53. The pharmaceutical formulation of item 47, wherein the non-polymeric lubricant is poorly water soluble or water insoluble and/or crystalline prior to compounding with said active pharmaceutical ingredient.

## Claims

1. A method of making a pharmaceutical composition comprising:
(a) providing an active pharmaceutical ingredient and one or more pharmaceutically acceptable excipients including a non-polymeric lubricant, and wherein the non-polymeric lubricant is selected from the group consisting of magnesium stearate, calcium stearate, (sodium) stearyl fumarate, glyceryl monostearate, glyceryl palmitostearate, myristic acid, palmitic acid, or zinc stearate;
(b) processing the materials of step (a) using thermal processing or solvent evaporation,
wherein the processing forms a pharmaceutical composition comprising a single phase amorphous solid dispersion of the active pharmaceutical ingredient and the one or more pharmaceutically acceptable excipients including the non-polymeric lubricant, wherein the non-polymeric lubricant is in an amorphous state.

2. The method of claim 1, wherein said pharmaceutical comprises more than one active pharmaceutical ingredient.

3. The method of claim 1, wherein the one or more pharmaceutically acceptable excipient comprises
a surfactant, preferably wherein the surfactant comprises an agent selected from the group consisting of sodium dodecyl sulfate, dioctyl sodium sulphosuccinate, polyoxyethylene (20) sorbitan monooleate, glycerol polyethylene glycol oxystearate-fatty acid glycerol polyglycol esters-polyethylene glycols-glycerol ethoxylate, glycerol-polyethylene glycol ricinoleate-fatty acid esters of polyethyleneglycol-polyethylene glycols-ethoxylated glycerol, vitamin E TPGS, and sorbitan laurate, and a pharmaceutical polymer selected from the group consisting of poly(vinylpyrrolidone), ethylacrylate-methylmethacrylate copolymer, poly(methacrylate ethylacrylate) (1:1) copolymer, hydroxypropylmethylcellulose acetate succinate, poly(butyl methacylate-co-(2-dimethylaminoethyl) methacrylate-co-methyl methacrylate) 1:2:1 and polyvinyl caprolactam-polyvinyl acetate-polyethylene glycol graft copolymer, or
a pharmaceutical polymer selected from the group consisting of poly(vinyl acetate)-co-poly(vinylpyrrolidone) copolymer, ethylcellulose, hydroxypropylcellulose, cellulose acetate butyrate, poly(vinylpyrrolidone), poly(ethylene glycol), poly(ethylene oxide), poly(vinyl alcohol), hydroxypropyl methylcellulose, ethylcellulose, hydroxyethylcellulose, sodium carboxymethyl-cellulose, dimethylaminoethyl methacrylate-methacrylic acid ester copolymer, ethylacrylate-methylmethacrylate copolymer, cellulose acetate phthalate, cellulose acetate trimelletate, poly(vinyl acetate) phthalate, hydroxypropylmethylcellulose phthalate, poly(methacrylate ethylacrylate) (1:1) copolymer, poly(methacrylate methylmethacrylate) (1:1) copolymer, poly(methacrylate methylmethacrylate) (1:2) copolymer, hydroxypropylmethylcellulose acetate succinate and polyvinyl caprolactam-polyvinyl acetate-polyethylene glycol graft copolymer, carbomer, crospovidone, or croscarmellose sodium, or
one or more surfactants and one or more polymer carriers, or
an agent selected from the group consisting of poly(vinyl acetate)-co-poly(vinylpyrrolidone) copolymer, ethylcellulose, hydroxypropylcellulose, cellulose acetate butyrate, poly(vinylpyrrolidone), poly(ethylene glycol), poly(ethylene oxide), poly(vinyl alcohol), hydroxypropyl methylcellulose, ethylcellulose, hydroxyethylcellulose, sodium carboxymethyl-cellulose, dimethylaminoethyl methacrylate-methacrylic acid ester copolymer, ethylacrylate-methylmethacrylate copolymer, cellulose acetate phthalate, cellulose acetate trimelletate, poly(vinyl acetate) phthalate, hydroxypropylmethylcellulose phthalate, poly(methacrylate ethylacrylate) (1:1) copolymer, poly(methacrylate methylmethacrylate) (1:1) copolymer, poly(methacrylate methylmethacrylate) (1:2) copolymer, hydroxypropylmethylcellulose acetate succinate and polyvinyl caprolactam-polyvinyl acetate-polyethylene glycol graft copolymer, carbomer, crospovidone, croscarmellose sodium, sodium dodecyl sulfate, dioctyl sodium sulphosuccinate, polyoxyethylene (20) sorbitan monooleate, glycerol polyethylene glycol oxystearate-fatty acid glycerol polyglycol esters-polyethylene glycols-glycerol ethoxylate, glycerol-polyethylene glycol ricinoleate-fatty acid esters of polyethyleneglycol-polyethylene glycols-ethoxylated glycerol, vitamin E TPGS and sorbitan laurate, or
a processing agent, such as a plasticizer, or
a pharmaceutical polymer of high melt viscosity, or
a thermally labile pharmaceutical polymer.

4. The method of claim 1, wherein step (b) is performed at a maximum temperature of 250 °C, 225 °C, 200 °C, 180 °C, °C or 150 °C to 250 °C.

5. The method of claims 1-4, wherein thermal processing comprises hot melt extrusion or thermokinetic processing.

6. The method of claim 1, wherein the active pharmaceutical ingredient to pharmaceutical excipient ratio is 1 to 4, 3 to 7, 2 to 3 or 1 to 1.

7. The method of claims 1-6, wherein the non-polymeric lubricant is poorly water soluble or water insoluble and/or crystalline prior to compounding with said active pharmaceutical ingredient.

8. A pharmaceutical composition comprising a single phase amorphous solid dispersion of (a) an active pharmaceutical ingredient, (b) one or more pharmaceutically acceptable excipients, and (c) a non-polymeric lubricant selected from the group consisting of magnesium stearate, calcium stearate, (sodium) stearyl fumarate, glyceryl monostearate, glyceryl palmitostearate, myristic acid, palmitic acid, or zinc stearate, wherein the non-polymeric lubricant is in an amorphous state.

9. The pharmaceutical composition of claim 8, wherein said pharmaceutical comprises more than one active pharmaceutical ingredient.

10. The pharmaceutical composition of claim 8, wherein the one or more pharmaceutically acceptable excipient comprises
a surfactant, preferably wherein the surfactant comprises an agent selected from the group consisting of sodium dodecyl sulfate, dioctyl sodium sulphosuccinate, polyoxyethylene (20) sorbitan monooleate, glycerol polyethylene glycol oxystearate-fatty acid glycerol polyglycol esters-polyethylene glycols-glycerol ethoxylate, glycerol-polyethylene glycol ricinoleate-fatty acid esters of polyethyleneglycol-polyethylene glycols-ethoxylated glycerol, vitamin E TPGS, and sorbitan laurate, and
a pharmaceutical polymer selected from the group consisting of poly(vinylpyrrolidone), hydroxypropylcellulose, poly(vinyl alcohol), hydroxypropyl methylcellulose, hydroxyethylcellulose, and sodium carboxymethyl-cellulose.and polyvinyl caprolactam-polyvinyl acetate-polyethylene glycol graft copolymer, of poly(vinyl acetate)-co-poly(vinylpyrrolidone) copolymer, ethylcellulose, hydroxypropylcellulose, cellulose acetate butyrate, poly(vinylpyrrolidone), poly(ethylene glycol), poly(ethylene oxide), poly(vinyl alcohol), hydroxypropyl methylcellulose, ethylcellulose, hydroxyethylcellulose, sodium carboxymethyl-cellulose, dimethylaminoethyl methacrylate-methacrylic acid ester copolymer, ethylacrylate-methylmethacrylate copolymer, cellulose acetate phthalate, cellulose acetate trimelletate, poly(vinyl acetate) phthalate, hydroxypropylmethylcellulose phthalate, poly(methacrylate ethylacrylate) (1:1) copolymer, poly(methacrylate methylmethacrylate) (1:1) copolymer, poly(methacrylate methylmethacrylate) (1:2) copolymer, hydroxypropylmethylcellulose acetate succinate and polyvinyl caprolactam-polyvinyl acetate-polyethylene glycol graft copolymer, carbomer, crospovidone, or croscarmellose sodium, or
a plasticizer, or
wherein the pharmaceutically acceptable excipient comprises an agent selected from the group consisting of poly(vinyl acetate)-co-poly(vinylpyrrolidone) copolymer, ethylcellulose, hydroxypropylcellulose, cellulose acetate butyrate, poly(vinylpyrrolidone), poly(ethylene glycol), poly(ethylene oxide), poly(vinyl alcohol), hydroxypropyl methylcellulose, ethylcellulose, hydroxyethylcellulose, sodium carboxymethyl-cellulose, dimethylaminoethyl methacrylate-methacrylic acid ester copolymer, ethylacrylate-methylmethacrylate copolymer, cellulose acetate phthalate, cellulose acetate trimelletate, poly(vinyl acetate) phthalate, hydroxypropylmethylcellulose phthalate, poly(methacrylate ethylacrylate) (1:1) copolymer, poly(methacrylate methylmethacrylate) (1:1) copolymer, poly(methacrylate methylmethacrylate) (1:2) copolymer, hydroxypropylmethylcellulose acetate succinate, polyvinyl caprolactam-polyvinyl acetate-polyethylene glycol graft copolymer, carbomer, crospovidone, croscarmellose sodium, sodium dodecyl sulfate, dioctyl sodium sulphosuccinate, polyoxyethylene (20) sorbitan monooleate, glycerol polyethylene glycol oxystearate-fatty acid glycerol polyglycol esters-polyethylene glycols-glycerol ethoxylate, glycerol-polyethylene glycol ricinoleate-fatty acid esters of polyethyleneglycol-polyethylene glycols-ethoxylated glycerol, vitamin E TPGS, and sorbitan laurate, or
wherein the one or more pharmaceutically acceptable excipients comprises a pharmaceutical polymer of high melt viscosity, or
a thermally labile pharmaceutical polymer, or
wherein said pharmaceutical composition does not contain a processing agent, or
wherein said pharmaceutical composition does not contain a plasticizer, or
wherein the active pharmaceutical ingredient to pharmaceutical excipient ratio is 1 to 4, 3 to 7, 2 to 3, or 1 to 1.

11. The pharmaceutical composition of claim 8, wherein peak solubility of the active pharmaceutical ingredient and a reference standard of the active pharmaceutical ingredient in an aqueous buffer with a pH range of 4 to 8 have a ratio of greater than 3:1, greater than 10:1, greater than 20:1, or greater than 30:1, or
wherein Cₘₐₓ of the active pharmaceutical ingredient in the composition and Cₘₐₓ of the reference standard of the active pharmaceutical ingredient have a ratio that is greater than 6:1.

12. The pharmaceutical composition of claim 8, formulated into an oral dosage form, preferably wherein the oral dosage form is a tablet, a capsule, or a sachet.

13. The pharmaceutical composition of claims 8-12, wherein the non-polymeric lubricant is poorly water soluble or water insoluble and/or crystalline prior to compounding with said active pharmaceutical ingredient.

14. The pharmaceutical composition of claim 8 produced by a process comprising the steps of:
(a) providing the active pharmaceutical ingredient and the one or more pharmaceutically acceptable excipients including a non-polymeric lubricant;
(b) processing the materials of step (a) using thermal processing or solvent evaporation
wherein the processing forms the pharmaceutical composition comprising a single phase amorphous solid dispersion of the active pharmaceutical ingredient and the one or more pharmaceutically acceptable excipients including the non-polymeric lubricant.

15. The pharmaceutical composition of claim 14, wherein said one or more pharmaceutically acceptable excipients comprises an agent selected from the group consisting of poly(vinyl acetate)-co-poly(vinylpyrrolidone) copolymer, ethylcellulose, hydroxypropylcellulose, cellulose acetate butyrate, poly(vinylpyrrolidone), poly(ethylene glycol), poly(ethylene oxide), poly(vinyl alcohol), hydroxypropyl methylcellulose, ethylcellulose, hydroxyethylcellulose, sodium carboxymethyl-cellulose, dimethylaminoethyl methacrylate-methacrylic acid ester copolymer, ethylacrylate-methylmethacrylate copolymer, cellulose acetate phthalate, cellulose acetate trimelletate, poly(vinyl acetate) phthalate, hydroxypropylmethylcellulose phthalate, poly(methacrylate ethylacrylate) (1:1) copolymer, poly(methacrylate methylmethacrylate) (1:1) copolymer, poly(methacrylate methylmethacrylate) (1:2) copolymer, hydroxypropylmethylcellulose acetate succinate and polyvinyl caprolactam-polyvinyl acetate-polyethylene glycol graft copolymer sodium dodecyl sulfate, dioctyl sodium sulphosuccinate, polyoxyethylene (20) sorbitan monooleate, glycerol polyethylene glycol oxystearate-fatty acid glycerol polyglycol esters-polyethylene glycols-glycerol ethoxylate, glycerol-polyethylene glycol ricinoleate-fatty acid esters of polyethyleneglycol-polyethylene glycols-ethoxylated glycerol, vitamin E TPGS and sorbitan laurate, or
wherein said pharmaceutical composition comprises a processing agent, such as a plasticizer, or
wherein thermal processing comprises hot melt extrusion or thermokinetic processing, or
wherein the non-polymeric lubricant is poorly water soluble or water insoluble and/or crystalline prior to compounding with said active pharmaceutical ingredient.
